(19) Europäisches Patentamt / European Patent Office / Office européen des brevets

(11) **EP 4 606 907 A1**

(12) **EUROPEAN PATENT APPLICATION**
published in accordance with Art. 153(4) EPC

(43) Date of publication:
**27.08.2025 Bulletin 2025/35**

(21) Application number: **23879775.7**

(22) Date of filing: **17.10.2023**

(51) International Patent Classification (IPC):
*C12Q 1/6806* (2018.01)    *C12M 1/00* (2006.01)
*C12M 1/34* (2006.01)    *C12N 9/22* (2006.01)
*C12N 15/10* (2006.01)    *C12Q 1/34* (2006.01)
*C12Q 1/6876* (2018.01)    *G01N 1/10* (2006.01)
*G01N 1/40* (2006.01)

(52) Cooperative Patent Classification (CPC):
C12M 1/00; C12M 1/34; C12N 9/22; C12N 15/10;
C12Q 1/34; C12Q 1/6806; C12Q 1/6876;
G01N 1/10; G01N 1/40

(86) International application number:
**PCT/JP2023/037489**

(87) International publication number:
**WO 2024/085132 (25.04.2024 Gazette 2024/17)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.10.2022 JP 2022166631**

(71) Applicant: **Japan Science and Technology Agency
Kawaguchi-shi, Saitama 332-0012 (JP)**

(72) Inventors:
• **NOJI, Hiroyuki
Tokyo 113-8654 (JP)**
• **MINAGAWA, Yoshihiro
Tokyo 113-8654 (JP)**

(74) Representative: **Grünecker Patent- und
Rechtsanwälte
PartG mbB
Leopoldstraße 4
80802 München (DE)**

Remarks:
The complete document including Reference
Table(s) and the Sequence Listing(s) can be
downloaded from the EPO website

(54) **BIOLOGICAL SUBSTANCE TREATMENT METHOD, REACTION DETECTION METHOD, BIOLOGICAL SUBSTANCE TREATMENT APPARATUS, AND REACTION DETECTION APPARATUS**

(57) In a biological substance treatment method of the present invention, a flow cell 1 is prepared, the flow cell 1 including: a chamber array device 11 including a plurality of chambers 14, one of which is 1 nanoliter or less; and a cover body 21 defining a flow path 40 communicating with an opening of the chamber 14 and provided in common for the plurality of chambers 14. Next, DEX and PEG that are aqueous to each other and separate into phases when left to stand at room temperature are prepared. Here, DEX has a property of phase separation to the chamber 14 side, and has a property that the biological substance is preferentially distributed to DEX rather than PEG. Subsequently, the biological substance is mixed with PEG, and DEX is caused to flow into the flow path 40 to fill the chamber 14 and the flow path 40 with DEX. Next, PEG is caused to flow into the flow path 40 to push out the remaining DEX, and the biological substance is enriched in DEX in the chamber 14 by bringing DEX into contact with PEG.

EP 4 606 907 A1

[FIG. 7]

**Description**

Technical Field

The present invention relates to a biological substance treatment method, a reaction detection method, a biological substance treatment apparatus, and a reaction detection apparatus.

Methods and apparatuses for detecting a reaction of a biological substance have been developed in various fields such as academic research and medical care. A digital bioassay has emerged as a method of binarizing a signal from a microcompartment encapsulating an enzyme or enzyme-labeled molecule and performing quantification with single-molecule detection sensitivity. A single-molecule enzyme assay by microcompartmentalization was first reported in the 1960's, but a quantitative digital bioassay has become possible since microfabrication techniques have enabled the generation of micron-sized and uniformly shaped compartments. So far, the production of microcompartments of various types, such as droplets generated by flow focusing and hydrogel particle template droplets, has been reported. In accordance with the spread of these microcompartmentalization techniques, various enzyme assays can be digitized, and not only simple enzymes but also nucleic acids, antigens, viruses, membrane transport transporters, and the like can be measured.

The greatest feature of the digital bioassay method is high detection sensitivity as well as quantitativity that allows a concentration of a target molecule to be easily determined to within several orders of magnitude. Therefore, a digitized bioassay such as a digital enzyme immunoassay (digital ELISA) is expected as a next-generation diagnostic test.

In the digital bioassay, a chip in which a plurality of microspaces (chambers) are formed is used, and a biological reaction is detected using the chamber as a reaction field (reactor). A limit of detection (LOD) of the digital bioassay is limited by a total number of reactors x a volume of each reactor. Since three molecules are required to detect one or more positive reactors with a detection probability greater than or equal to 95% from all reactors, a concentration corresponding to three molecules per total reactor volume is an LOD of a practical device. A simple method to improve the LOD is to increase the total number of reactors or the volume of the reactors. However, there are physical restrictions on the size or scale of a microcompartment of an array-type microreactor or a microdroplet device.

Therefore, when the required detection sensitivity is higher than the theoretical LOD defined by the total reactor volume, an off-chip enrichment process is often employed as a pretreatment in digital bioassay. However, the off-chip enrichment process requires a step of handling a solution and adding equipment such as a centrifuge or a solution dispensing apparatus. On the other hand, an on-chip enrichment using dielectrophoresis or a magnetic field has been reported, but these methods require an external device such as a magnet or a power source.

The present inventors have developed a method for detecting minute substances contained in a plurality of receptacles formed to be separated from each other (see, for example, Patent Literature 1). This literature describes that a solvent containing a minute substance is introduced into a space between a lower layer portion and an upper layer portion in which a receptacle is formed, and a gas is introduced into the space to form a droplet of the solvent containing the minute substance in the receptacle. The receptacle has a bottom surface having a diameter of about 0.1 $\mu$m to 10 $\mu$m, a height (depth) of about 0.1 $\mu$m to 10 $\mu$m, and a volume of about 1 zeptoliter to 1 attoliter.

In addition, a technique for realizing multiplexing of a homogeneous immunoassay by an aqueous two-phase system is also known (see, for example, Non Patent Literature 1). This literature utilizes a micro-patterned aqueous two-phase system (ATPS) formed between polyethylene glycol (PEG) and dextran (DEX), which are phase-separating polymers. A custom 96-well microplate for Multiplex ATPS-AlphaLISA was used and ATPS consisting of 18 wt% PEG (Mw. 35 kDa) and 18 wt% DEX (Mw. 10 kDa) was used in an ATPS-multiplex homogeneous assay. The microplate uses white individual DEX microbasins (2.5 mm x 2.5 mm x 2.5 mm) arranged in a plate matrix (well size 8 mm × 8 mm, height 11.5 mm). In addition, in the presence of ATPS, a detection antibody, an acceptor bead, and a donor bead remain localized to the DEX droplets, indicating that the antigen is diffused from the bulk PEG phase to the DEX droplet.

The present literature describes that the PEG-antigen mixed liquid is prepared with acceptor beads for 4 types of antigens (CXCL10, CXCL9, IL-8, and IL-6) and biotinylated detection antibody 18% DEX, and the PEG-antigen mixed liquid was dispensed into a 384-well microplate so that each well contains about 2 $\mu$l of a standard sample and 8 $\mu$l of PEG (page 181). In addition, the present literature also describes that 100 $\mu$L of a PEG-antigen mixture is dispensed into a common PEG well, and a DEX solution is dispensed into each of four DEX microbasins using a multi-pipette.

Citation List

Patent Literature

Patent Literature 1: WO 2018/181488 A (claim 1, paragraph 0022, and the like)

Non Patent Literature

Non Patent Literature 1: Arlyne B. Simon et.al.; "Aqueous two-phase systems enable multiplexing of homogeneous immunoassays". May 2014, TECHNOLOGY 2(2):176-184, DOI:10.1142/S2339547814500150

Summary of Invention

Technical Problem

In Non Patent Literature 1, the size of the microbasin of the microplate is 2.5 mm × 2.5 mm × 2.5 mm (that is, 2.5 μl), and only four basins are provided. As will be appreciated, the microplate of the present literature is not intended for use in a digital bioassay in which a biological substance consisting of one or a small number of molecules is retained in a large number of wells to binarize reaction signals.

In addition, in the method of the present literature, first, the PEG-antigen mixture is dispensed into a common PEG well, and then, a DEX solution containing an acceptor bead and a detection antibody is dispensed into a DEX microbasin using a multi-pipette. When PEG and DEX are mixed by such a method, in a case where PEG or DEX exceeds a critical concentration, PEG and DEX are spontaneously phase-separated into an upper layer and a lower layer, respectively. For this reason, when the DEX solution is directly dispensed into the DEX microbasin with a pipette, mixing of PEG and DEX becomes insufficient, a reaction between an antigen in PEG and a detection reagent in DEX cannot be sufficiently performed, and it is difficult to detect the antigen. Furthermore, in the present literature, since the DEX solution is dispensed into each microbasin using the multi-pipette, it takes time and effort.

An object of the present invention is to provide a biological substance treatment method and a biological substance treatment apparatus capable of retaining a biological substance at a high concentration in a plurality of chambers by a simple operation even in a case where one of wells (hereinafter, a chamber) has a minute size of 1 nanoliter or less in a digital bioassay. Another object of the present invention is to provide a reaction detection method and a reaction detection apparatus capable of detecting a reaction of a biological substance with high sensitivity by such a simple operation.

Solution to Problem

An embodiment of the present invention is as follows.

[1] A biological substance treatment method for retaining and/or enriching a biological substance, the biological substance treatment method including:

a flow cell preparation step of preparing a flow cell including a chamber array device including a plurality of chambers each having a recess opened at one end, one of which is 1 nanoliter or less, and a cover defining a flow path communicating with the opening of the chamber and provided in common for the plurality of chambers;
a solvent preparation step of preparing a first solvent and a second solvent that are aqueous to each other and separate into phases when left to stand at room temperature, the first solvent having a property of separating to the chamber side when phase separation occurs in a region surrounded by the chamber and the flow path of the flow cell, and the first solvent and the second solvent having a property that the biological substance is preferentially distributed to the first solvent rather than the second solvent;
a mixing step of mixing the biological substance with the first solvent and/or the second solvent;
a first inflow step of causing one solvent of the first solvent and the second solvent to flow into the flow path to fill the chamber and the flow path with the solvent; and
a second inflow step of causing the other solvent of the first solvent and the second solvent to flow into the flow path to phase-separate the first solvent into the chamber side and the second solvent into the flow path side, retaining, in the chamber, the biological substance contained in the first solvent in the chamber without diffusing into the second solvent, and/or moving the biological substance contained in the second solvent into the first solvent in the chamber to enrich the biological substance.

In an embodiment of the present invention, since the plurality of chambers, one of which is 1 nanoliter or less, are included, it can be suitably used for a digital bioassay that retains and binarizes one molecule of the biological substance. In addition, one solvent of the first solvent and the second solvent is caused to flow into the flow path as a preceding solvent to be retained in the chamber, and then, the other solvent of the first solvent and the second solvent is caused to flow into the flow path to push out the preceding solvent remaining in the flow path. Therefore, the solvent can be retained in the chamber by a single simple operation without using a pipette or the like. Furthermore, since the first solvent and the second solvent are sufficiently in contact with each other, the biological substance is enriched in the first solvent in the chamber at a high concentration.

[2] The biological substance treatment method according to [1], in which the first solvent and the second solvent are selected from combinations indicated by Nos. 1 to 12 in Table 1.

[Table 1]

| No. | First solvent | Second solvent |
|---|---|---|
| 1 | Dextran aqueous solution | Polypropylene glycol aqueous solution |
| 2 | Polyethylene glycol aqueous solution | Polypropylene glycol aqueous solution |
| 3 | Dextran sulfate aqueous solution | Polyethylene glycol aqueous solution |
| 4 | Dextran sulfate aqueous solution | Carboxymethylcellulose aqueous solution |
| 5 | Polyethylene glycol aqueous solution | Phosphoric acid aqueous solution |
| 6 | Polyethylene glycol aqueous solution | Ficoll aqueous solution |
| 7 | Polyethylene glycol aqueous solution | Gelatin aqueous solution |
| 8 | polyU aqueous solution | Spermine aqueous solution |
| 9 | RGG domain + NaCl aqueous solution | Water |
| 10 | DDX4 N-terminal domain + NaCl aqueous solution | Water |
| 11 | Prion-like domain + NaCl aqueous solution | Water |
| 12 | Dextran aqueous solution | Aqueous solution containing copolymer of polyethylene glycol and polypropylene glycol |

As described above, a solvent can be selected from a plurality of combinations of the first solvent and the second solvent and then used.

[3] The biological substance treatment method according to [2], in which the first solvent is a dextran aqueous solution, and the second solvent is a polyethylene glycol aqueous solution.

As described above, the first solvent (dextran) can be retained in the chamber by using the dextran aqueous solution and the polyethylene glycol aqueous solution having a property of being easily phase-separated.

[4] The biological substance treatment method according to [1], further including a tag adding step of adding a tag that improves a distribution property to the first solvent to the biological substance.

As described above, as the tag that improves the distribution property to the first solvent to the biological substance is added, even a biological substance originally having a low distribution property to the first solvent can be easily distributed and retained in the first solvent.

[5] The biological substance treatment method according to [4], in which the tag is selected from the group consisting of a dextran binding domain derived from a lactic acid bacterium *"Leuconostoc mesenteroides"*, dextran, a dextran-like molecule polymer, DNA, RNA, a chemically modified molecule thereof, and a nucleic acid-like molecule polymer.

By using such a tag, the distribution property of the biological substance to the first solvent (particularly, dextran) can be improved.

[6] The biological substance treatment method according to [1], further including, after the second inflow step, a sealing solvent inflow step of causing a sealing solvent to flow into the flow path to push out the solvent remaining in the flow path from the flow path.

As described above, the first solvent is prevented from flowing out of the chamber by sealing the flow path with the sealing solvent.

[7] A reaction detection method including a detection step of detecting a reaction of the biological substance retained and/or enriched by the biological substance treatment method according to [1].

According to the present embodiment, the reaction of the biological substance enriched in the chamber at a high concentration can be detected with high sensitivity by a simple operation.

[8] The reaction detection method according to [7], in which the biological substance includes at least:

a target RNA;
crRNA having a sequence complementary to a specific region of the target RNA;

Cas13 that forms a complex with the crRNA, the Cas13 cleaving the target RNA when the crRNA binds to the target RNA as a guide RNA to form an activated complex;

a first RNA probe having a sequence capable of being cleaved by the activated complex and binding a first fluorescent dye, the first RNA probe emitting a first fluorescence by being cleaved by the activated complex or a sequence non-specific ribonuclease; and

a second RNA probe that does not have a sequence to be cleaved by the activated complex, the second RNA probe binding a second fluorescent dye that emits fluorescence distinguishable from the first fluorescent dye and emitting a second fluorescence by being cleaved by the sequence non-specific ribonuclease, and

the detection step includes:

a positive measurement step of measuring a chamber emitting only the first fluorescence as a positive signal; and

a false positive measurement step of measuring a chamber emitting both the first fluorescence and the second fluorescence as false positive signals by the sequence non-specific ribonuclease.

As described above, it is possible to more accurately detect the target RNA by distinguishing between the positive signal and the false positive signal by using at least two types of probes.

[9] A biological substance treatment apparatus for retaining and/or enriching a biological substance, the biological substance treatment apparatus including:

a flow cell including a chamber array device including a plurality of chambers each having a recess opened at one end, one of which is 1 nanoliter or less, and a cover defining a flow path communicating with the opening of the chamber and provided in common for the plurality of chambers;

a first solvent and a second solvent that are aqueous to each other and separate into phases when left to stand at room temperature, the first solvent having a property of separating to the chamber side when phase separation occurs in a region surrounded by the chamber and the flow path of the flow cell, and the first solvent and the second solvent having a property that the biological substance is preferentially distributed to the first solvent rather than the second solvent;

means for mixing the biological substance with the first solvent and/or the second solvent;

first inflow means for causing one solvent of the first solvent and the second solvent to flow into the flow path to fill the chamber and the flow path with the solvent; and

second inflow means for causing the other solvent of the first solvent and the second solvent to flow into the flow path to phase-separate the first solvent into the chamber side and the second solvent into the flow path side, retaining, in the chamber, the biological substance contained in the first solvent in the chamber without diffusing into the second solvent, and/or moving the biological substance contained in the second solvent into the first solvent in the chamber to enrich the biological substance.

In an embodiment of the present invention, since the plurality of chambers, one of which is 1 nanoliter or less, are included, it can be suitably used for a digital bioassay that retains and binarizes one molecule of the biological substance. In addition, one of the first solvent and the second solvent is caused to flow into the flow path as a preceding solvent to be retained in the chamber, and then, the other of the first solvent and the second solvent is caused to flow into the flow path to push out the preceding solvent remaining in the flow path. Therefore, the solvent can be retained in the chamber by a single simple operation without using a pipette or the like. Furthermore, since the first solvent and the second solvent are sufficiently in contact with each other, the biological substance is enriched in the first solvent in the chamber at a high concentration.

[10] The biological substance treatment apparatus according to [9], in which the first solvent and the second solvent are selected from combinations indicated by Nos. 1 to 12 in Table 2.

[Table 2]

| No. | First solvent | Second solvent |
| --- | --- | --- |
| 1 | Dextran aqueous solution | Polypropylene glycol aqueous solution |
| 2 | Polyethylene glycol aqueous solution | Polypropylene glycol aqueous solution |
| 3 | Dextran sulfate aqueous solution | Polyethylene glycol aqueous solution |
| 4 | Dextran sulfate aqueous solution | Carboxymethylcellulose aqueous solution |
| 5 | Polyethylene glycol aqueous solution | Phosphoric acid aqueous solution |
| 6 | Polyethylene glycol aqueous solution | Ficoll aqueous solution |
| 7 | Polyethylene glycol aqueous solution | Gelatin aqueous solution |

(continued)

| No. | First solvent | Second solvent |
|---|---|---|
| 8 | polyU aqueous solution | Spermine aqueous solution |
| 9 | RGG domain + NaCl aqueous solution | Water |
| 10 | DDX4 N-terminal domain + NaCl aqueous solution | Water |
| 11 | Prion-like domain + NaCl aqueous solution | Water |
| 12 | Dextran aqueous solution | Aqueous solution containing copolymer of polyethylene glycol and polypropylene glycol |

As described above, a solvent can be selected from a plurality of combinations of the first solvent and the second solvent and then used.

[11] The biological substance treatment apparatus according to [10], in which the first solvent is a dextran aqueous solution, and the second solvent is a polyethylene glycol aqueous solution.

As described above, the first solvent (dextran) can be retained in the chamber by using the dextran aqueous solution and the polyethylene glycol aqueous solution having a property of being easily phase-separated.

[12] The biological substance treatment method according to [9], in which the biological substance includes a tag that improves a distribution property to the first solvent.

As described above, as the tag that improves the distribution property to the first solvent to the biological substance is added, even a biological substance originally having a low distribution property to the first solvent can be easily distributed and retained in the first solvent.

[13] The biological substance treatment apparatus according to [12], in which the tag is selected from the group consisting of a dextran binding domain derived from a lactic acid bacterium *"Leuconostoc mesenteroides",* dextran, a dextran-like molecule polymer, DNA, RNA, a chemically modified molecule thereof, and a nucleic acid-like molecule polymer.

By using such a tag, the distribution property of the biological substance to the first solvent (particularly, dextran) can be improved.

[14] The biological substance treatment apparatus according to claim [9], further including a sealing solvent.

As described above, the first solvent is prevented from flowing out of the chamber by sealing the flow path with the sealing solvent.

[15] A reaction detection apparatus further including detection means for detecting a reaction of the biological substance retained and/or enriched by the biological substance treatment apparatus according to [9].

According to the present embodiment of the present invention, the reaction of the biological substance enriched in the chamber at a high concentration can be detected with high sensitivity by a simple operation.

[16] The reaction detection apparatus according to [15], in which the biological substance includes at least:

a target RNA;

crRNA having a sequence complementary to a specific region of the target RNA;

Cas13 that forms a complex with the crRNA, the Cas13 cleaving the target RNA when the crRNA binds to the target RNA as a guide RNA to form an activated complex;

a first RNA probe having a sequence capable of being cleaved by the activated complex and binding a first fluorescent dye, the first RNA probe emitting a first fluorescence by being cleaved by the activated complex or a sequence non-specific ribonuclease; and

a second RNA probe that does not have a sequence to be cleaved by the activated complex, the second RNA probe binding a second fluorescent dye that emits fluorescence distinguishable from the first fluorescent dye and emitting a second fluorescence by being cleaved by the sequence non-specific ribonuclease, and

the detection means includes:

positive measurement means for measuring a chamber emitting only the first fluorescence as a positive signal; and

false positive measurement means for measuring a chamber emitting both the first fluorescence and the second fluorescence as false positive signals by the sequence non-specific ribonuclease.

As described above, it is possible to more accurately detect the target RNA by distinguishing between the positive signal and the false positive signal by using at least two types of probes.

[17] A biological substance treatment apparatus for retaining and/or enriching a biological substance using a flow cell including a chamber array device including a plurality of chambers each having a recess opened at one end, one of which is 1 nanoliter or less, and a flow path communicating with the opening of the chamber and provided in common for the plurality of chambers, the biological substance treatment apparatus including:

a first solvent and a second solvent that are aqueous to each other and separate into phases when left to stand at room temperature, the first solvent having a property of separating to the chamber side when phase separation occurs in a region surrounded by the chamber and the flow path of the flow cell, and the first solvent and the second solvent having a property that the biological substance is preferentially distributed to the first solvent rather than the second solvent;

means for mixing the biological substance with the first solvent and/or the second solvent;

first inflow means for causing one of the first solvent and the second solvent to flow into the flow path to fill the chamber and the flow path with the solvent; and

second inflow means for causing the other of the first solvent and the second solvent to flow into the flow path to phase-separate the first solvent into the chamber side and the second solvent into the flow path side, retaining, in the chamber, the biological substance contained in the first solvent in the chamber without diffusing into the second solvent, and/or moving the biological substance contained in the second solvent into the first solvent in the chamber to enrich the biological substance.

[Another technical feature 1] A reaction detection method for detecting a target RNA, the reaction detection method including:

a step of preparing:

a target RNA;

crRNA having a sequence complementary to a specific region of the target RNA;

Cas13 that forms a complex with the crRNA, the Cas13 cleaving the target RNA when the crRNA binds to the target RNA as a guide RNA to form an activated complex;

a first RNA probe having a sequence capable of being cleaved by the activated complex and binding a first fluorescent dye, the first RNA probe emitting a first fluorescence by being cleaved by the activated complex or a sequence non-specific ribonuclease; and

a second RNA probe that does not have a sequence to be cleaved by the activated complex, the second RNA probe binding a second fluorescent dye that emits fluorescence distinguishable from the first fluorescent dye and emitting a second fluorescence by being cleaved by the sequence non-specific ribonuclease;

a reaction step of allowing a cleavage reaction of the target RNA by the complex and an accompanying cleavage reaction of the first RNA probe to proceed, and/or allowing a cleavage reaction of the first RNA probe and the second RNA probe by a sequence non-specific ribonuclease contained in an environment to proceed;

a positive measurement step of measuring a positive signal when only the first fluorescence is emitted; and

a false positive measurement step of measuring a false positive signal by the sequence non-specific ribonuclease when both the first fluorescence and the second fluorescence are emitted.

[Another technical feature 2] A reaction detection apparatus for detecting a target RNA, the reaction detection apparatus including:

the target RNA;

crRNA having a sequence complementary to a specific region of the target RNA;

Cas13 that forms a complex with the crRNA, the Cas13 cleaving the target RNA when the crRNA binds to the target RNA as a guide RNA to form an activated complex;

a first RNA probe having a sequence capable of being cleaved by the activated complex and binding a first fluorescent dye, the first RNA probe emitting a first fluorescence by being cleaved by the activated complex or a sequence non-specific ribonuclease;

a second RNA probe that does not have a sequence to be cleaved by the activated complex, the second RNA probe binding a second fluorescent dye that emits fluorescence distinguishable from the first fluorescent dye and emitting a second fluorescence by being cleaved by the sequence non-specific ribonuclease;

reaction means for allowing a cleavage reaction of the target RNA by the complex and an accompanying cleavage reaction of the first RNA probe to proceed, and/or allowing a cleavage reaction of the first RNA probe and the second RNA probe by a sequence non-specific ribonuclease contained in an environment to proceed;

positive measurement means for measuring a positive signal when only the first fluorescence is emitted; and

false positive measurement means for measuring a false positive signal by the sequence non-specific ribonuclease when both the first fluorescence and the second fluorescence are emitted.

Advantageous Effects of Invention

According to the present invention, it is possible to provide a biological substance treatment method and a biological substance treatment retaining apparatus capable of retaining a biological substance in a plurality of chambers by a simple operation even in a case where the chamber has a minute size of 1 nanoliter or less in a digital bioassay. In addition, according to the present invention, it is possible to provide a reaction detection method and a reaction detection apparatus capable of detecting a reaction of a biological substance with high sensitivity by such a simple operation.

Brief Description of Drawings

Fig. 1 is a perspective exploded view illustrating an embodiment of a flow cell used in the present invention.
Fig. 2 is a side cross-sectional view of the flow cell.
Fig. 3 is a side cross-sectional view illustrating a procedure for enriching a biological substance using the flow cell.
Fig. 4 is a side cross-sectional view illustrating a procedure for enriching a biological substance using the flow cell.
Fig. 5 is a view illustrating a method for detecting a target RNA using Cas13.
Fig. 6 is an example of an image obtained by measuring fluorescence of a flow cell in a system using the Cas13.
Fig. 7 is a view illustrating an outline and a result of an experiment of a femtoliter DEX droplet array system in an embodiment.
Fig. 8 is a view illustrating a diameter and a volume of a DEX reactor formed in the femtoliter chamber array.
Fig. 9 is a view illustrating a binomial curve and an estimated tie line.
Fig. 10 is a view illustrating results of a digital bioassay of EcALP by the DEX droplet system of an Example.
Fig. 11 illustrates a digital RNA counting experiment by Cas13 using the DEX droplet system.
Fig. 12 is a graph showing experimental results of a dual reporter system that suppresses a false positive signal.
Fig. 13 is a view illustrating a model of an enrichment factor in a digital assay using DEX droplets.
Fig. 14 is a view illustrating an incubation period dependency of a concentration of ALP-DBD.
Fig. 15 is a view illustrating a result of an experiment in which a dextran reactor is formed in a chamber in which a ratio between a diameter and a depth is changed.
Fig. 16 is a view illustrating an outline and results of an antibody concentration experiment using a tag that improves a distribution ratio to DEX.
Fig. 17 is a view illustrating distribution coefficients of ALP and ALP-DBD to ATPS for DEX/PEG.
Fig. 18 is a view illustrating a result of an experiment in which a biological substance is placed only in DEX using DEX as a first solvent and PEG as a second solvent.
Fig. 19 is a view illustrating a result of an experiment in which PEG is used as a first solvent and DEX is used as a second solvent.
Fig. 20 is a view illustrating a result of an experiment in which PEG is used as a first solvent and phosphoric acid is used as a second solvent.
Fig. 21 is a conceptual view for explaining a system for enriching a target DNA by DBD-capturing DNA.

Description of Embodiments

1. Biological Substance Treatment Method

Hereinafter, a biological substance treatment method according to an embodiment of the present invention will be described. The present invention is a biological substance treatment method for retaining and/or enriching a biological substance, the biological substance treatment method including a flow cell preparation step, a solvent preparation step, a mixing step, and a first inflow step. Hereinafter, each step will be described in order. In the present embodiment, examples of an enzyme E and a substrate S are described as biological substances, but the present invention is not limited thereto, and can be applied to various biological substances.

(1) Flow Cell Preparation Step

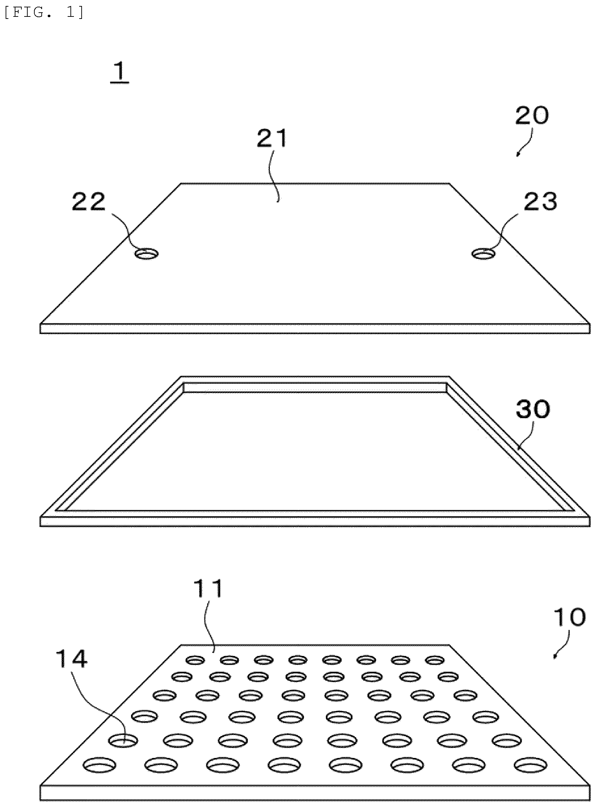

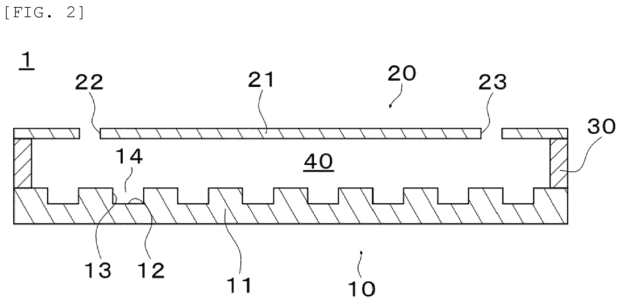

The reflow cell preparation step is a step of preparing a flow cell including a plurality of chambers, one of which is 1 nanoliter or less, and a flow path located above the plurality of chambers and through which a solvent flows.
A flow cell 1 used in the present embodiment will be described with reference to Figs. 1 and 2. Fig. 1 is an exploded perspective view of the flow cell 1, and Fig. 2 is a side cross-sectional view of the flow cell 1.
As illustrated in Fig. 1, the flow cell 1 is a chip including a base 10, a cover 20, and a spacer 30. The base 10 includes a chamber array device 11, and a plurality of chambers 14, which are minute spaces, are provided on a surface of the chamber array device 11. The chamber array device 11 is a plate-like member (substrate) formed of glass, an acrylic resin, or the like. The chamber 14 is a recess formed on the surface of the chamber array device 11 and opened at one end. As illustrated in Fig. 2, the chamber 14 is a space portion defined by a bottom surface 12 and a side surface 13. Each of the chambers 14 has a volume of 1 nanoliter or less, a first solvent is stored therein as described below, and the biological substance is enriched in the first solvent. When a reaction of one molecule of a biological substance is detected by a digital bioassay, the volume of the chamber 14 is approximately 10 picoliters or less, preferably 1 picoliter or less, more preferably 100 femtoliters or less, and particularly preferably 50 femtoliters or less, depending on a molecular weight of the biological substance. A lower limit of the chamber 14 is not particularly limited, and is preferably zeptoliter or more, and more preferably 1 attoliter or more. When a biological substance or the like is reacted inside the chamber, the chamber serves as a reactor, and therefore, in the present specification, the chamber may be referred to as a "reactor". The present invention also includes a case where the biological substance is not reacted in the chamber, for example, a case where the biological substance is simply enriched in the chamber and is not reacted, and a case where leakage of the biological substance from the inside of the chamber is prevented. In addition, when a biological substance or the like is reacted in such a reactor, the flow cell can also be referred to as a "reactor chip". In addition, in a flat-plate-like chip as in the present embodiment, the chamber can also be referred to as a "well". In addition, the shape of the chamber 14 is not limited to a hole shape as in the present embodiment, and may be another shape such as a groove. Further, a liquid droplet formed by aqueous two-layer separation is included as a chamber.

These drawings schematically illustrate the flow cell 1, and illustrate a view in which the number of chambers 14 is reduced for easy understanding. In the flow cell 1 actually used in an experiment or the like, the number of chambers 14 is about 500,000 to 5,000,000, for example, 1,000,000.

The chamber 14 can be formed on the surface of the chamber array device 11 by a microfabrication technique by photolithography or the like. In the processing method by photolithography, a chamber array device 11 coated with a photoresist such as a fluorine-based resin is prepared, a photomask having a target pattern formed thereon is arranged thereon, and the pattern of the photomask is transferred to the photoresist by irradiation with ultraviolet rays. Thereafter, the photoresist is etched with an etching solution to manufacture the chamber array device 11 in which a shape of a target pattern is formed.

The chamber 14 is preferably cylindrical as shown in the drawing. In addition, a diameter of the chamber 14 (a diameter of the bottom surface 12 in the drawing) is in a range of 1 to 100 $\mu$m, and preferably in a range of 5 to 20 $\mu$m. Further, a depth of the chamber 14 (a height of the side surface 13) is in a range of 1 to 100 $\mu$m, and preferably in a range of 5 to 20 $\mu$m. From the viewpoint of processing accuracy, the chamber 14 preferably has a ratio represented by "diameter (the diameter of the bottom surface 12 in the drawing)/depth (the height of the side surface 13)" of 5 or less, and more preferably 2 or less. In addition, from the viewpoint of retention of the solvent in the chamber 14, the ratio is preferably 1.5 or less. When the ratio is more than 1.5, the depth of the chamber 14 with respect to the diameter becomes shallow, and it becomes difficult to accommodate a solvent (dextran aqueous solution 50). In addition, when the ratio is more than 1.5, the dextran aqueous solution 50 in the chamber 14 is washed away by another solvent (polyethylene glycol aqueous solution 60), and it becomes difficult to retain the dextran aqueous solution 50 in the chamber 14. A lower limit of the ratio is not particularly limited, and is preferably 0.1 or more, and more preferably 0.5 or more.

As illustrated in Fig. 1, the cover 20 includes a cover body 21, a solvent inlet 22, and a solvent outlet 23. The cover body 21 is a plate-like member formed of glass, a resin, or the like. The solvent inlet 22 penetrating from an upper surface to a lower surface of the cover body 21 is provided in a portion of the cover body 21. In addition, the solvent outlet 23 penetrating from the upper surface to the lower surface of the cover body 21 is provided in the other portion of the cover body 21. These are openings for allowing the solvent to flow in and out, respectively.

The spacer 30 is provided between the base 10 and the cover 20, and is a member for sealing them over the entire side portion. The spacer 30 has a rectangular shape in a top view of an outer edge portion, and has a shape in which an opening portion is formed inside an outer edge portion. The spacer 30 is formed of a double-sided tape, rubber, or the like. Then, the flow path 40 is formed in a portion surrounded by an upper surface of the chamber array device 11 of the base 10, a lower surface of the cover body 21 of the cover 20, and an inner wall surface of the spacer 30. The flow path 40 is a sealed space that does not communicate with the outside except for the solvent inlet 22 and the solvent outlet 23. In addition, the flow path 40 is a space portion communicating with the opening of the recess constituting the chamber 14, and is a space portion communicating with all of the plurality of chambers 14 and provided in common to the plurality of chambers 14. In order to prevent adhesion of biological substances, the surface of the base 10 or the cover 20 facing the flow path 40 may be surface-treated in advance with a blocking solution such as a surfactant.

A volume of the flow path 40 can be appropriately set according to the properties of the solvent or the biological substance, and is, for example, in a range of 1 to 100 $\mu$l, preferably in a range of 5 to 50 $\mu$l, and more preferably in a range of 7 to 8 $\mu$l. A ratio of the total volume of the plurality of chambers 14 to the volume of the flow path 40 can be appropriately set, and is preferably within a range of 1:50 to 1:200, and particularly preferably about 1:100. By setting the volume ratio within this range, droplets can be efficiently formed in the chamber 14.

(2) Solvent Preparation Step

The solvent preparation step is a step of preparing two solvents (a first solvent and a second solvent) that are aqueous to each other. When these solvents are left to stand at normal temperature (30°C), the first solvent and the second solvent spontaneously phase-separated from each other when their respective concentrations exceed a critical concentration. In addition, the biological substance has a property that the biological substance is preferentially distributed to the first solvent rather than the second solvent. In other words, the biological substance is more likely to move to the first solvent than the second solvent. More specifically, it means that the concentration of the biological substance in the first solvent is higher than the concentration of the biological substance in the second solvent when the first solvent, the second solvent, and the biological substance are mixed, the first solvent and the second solvent are phase-separated, and the biological substance reaches distribution equilibrium. In other words, it can also be said that the "distribution coefficient" defined by the following formula exceeds 1.

Distribution coefficient = Concentration of biological substance dissolved in first solvent/Concentration of biological substance dissolved in second solvent when distribution equilibrium is reached

Here, the distribution coefficient is preferably 10 or more, and more preferably 50 or more. As the distribution coefficient is higher, the biological substance is preferentially moved into the first solvent in a state where the first solvent and the second solvent are phase-separated from each other, and is more easily enriched in the first solvent. In the present specification, the property that a biological substance is preferentially distributed to one of two solvents that are phase-separated may be expressed as a "distribution property".

When the specific gravity of the first solvent is larger than the specific gravity of the second solvent, the first solvent is separated into a lower layer and the second solvent is separated into an upper layer by phase separation.

The first solvent is preferably a dextran aqueous solution, and the second solvent is preferably a polyethylene glycol aqueous solution. The dextran aqueous solution is obtained by dissolving dextran in a good solvent such as water, methanol, ethanol, benzene, or chloroform. Similarly, the polyethylene glycol aqueous solution is obtained by dissolving polyethylene glycol in a good solvent such as water, methanol, ethanol, benzene, or chloroform. In the present embodiment, a dextran (DEX) aqueous solution is used as the first solvent, and a polyethylene glycol (PEG) aqueous solution is used as the second solvent.

A weight average molecular weight (MW) of dextran used in the first solvent can be appropriately set, and is, for example, in a range of 100,000 to 1,000,000, and preferably in a range of 300,000 to 700,000. When a dextran binding domain derived from a lactic acid bacterium *"Leuconostoc mesenteroides"* to be described below is added as a tag to a biological substance, dextran derived from the lactic acid bacterium is preferable. A concentration of dextran contained in the dextran aqueous solution 50 can be appropriately set, and can be, for example, within a range of 1 to 10 wt% (w/w), and more preferably within a range of 4 to 6 wt% (w/w).

A weight average molecular weight (MW) of polyethylene glycol used in the second solvent can be appropriately set, and is, for example, in a range of 10,000 to 100,000, and preferably in a range of 20,000 to 50,000. A concentration of polyethylene glycol contained in the polyethylene glycol aqueous solution 60 can be appropriately set, and can be, for example, within a range of 1 to 10% (w/w), and particularly preferably within a range of 4 to 6% (w/w). The dextran aqueous solution 50 and the polyethylene glycol aqueous solution 60 of the present embodiment have a concentration exceeding the critical concentration.

As the first solvent and the second solvent of the present embodiment, the following combinations can also be used. From the top to "polyU aqueous solution" in the following table are segregative (biased) LLPS (liquid-liquid phase separation), and the lower part of the table is coaservation (self-assembled) LLPS. Such a self-assembled LLPS includes not only the nucleic acid or the natural denatured protein exemplified in Nos. 1 to 12 in the following table but also various polymers.

[Table 3]

| No. | First solvent | Second solvent |
|---|---|---|
| 1 | Dextran aqueous solution | Polypropylene glycol aqueous solution |
| 2 | Polyethylene glycol aqueous solution | Polypropylene glycol aqueous solution |
| 3 | Dextran sulfate aqueous solution | Polyethylene glycol aqueous solution |
| 4 | Dextran sulfate aqueous solution | Carboxymethylcellulose aqueous solution |
| 5 | Polyethylene glycol aqueous solution | Phosphoric acid aqueous solution |
| 6 | Polyethylene glycol aqueous solution | Ficoll aqueous solution |
| 7 | Polyethylene glycol aqueous solution | Gelatin aqueous solution |

(continued)

| No. | First solvent | Second solvent |
|---|---|---|
| 8 | polyU aqueous solution | Spermine aqueous solution |
| 9 | RGG domain + NaCl aqueous solution | Water |
| 10 | DDX4 N-terminal domain + NaCl aqueous solution | Water |
| 11 | Prion-like domain + NaCl aqueous solution | Water |
| 12 | Dextran aqueous solution | Aqueous solution containing copolymer of polyethylene glycol and polypropylene glycol |

(3) Mixing Step

In the mixing step, the biological substance is mixed with the first solvent and/or the second solvent. That is, the biological substance may be mixed with the first solvent, may be mixed with the second solvent, or may be mixed with both solvents. In the present embodiment, the biological substance is not mixed with dextran as the first solvent, but the enzyme E as the biological substance and the substrate S are mixed with polyethylene glycol as the second solvent and used.

(4) First Inflow Step

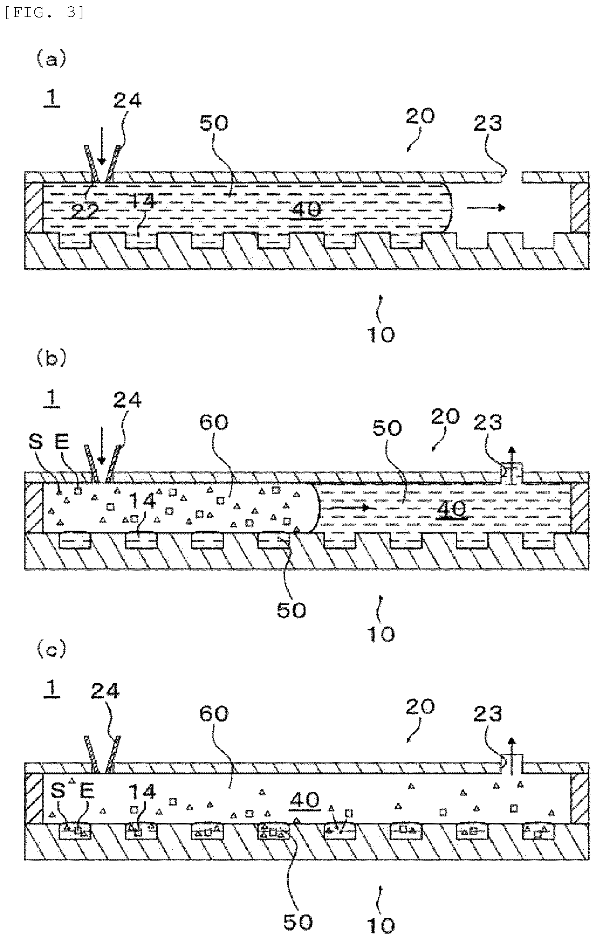

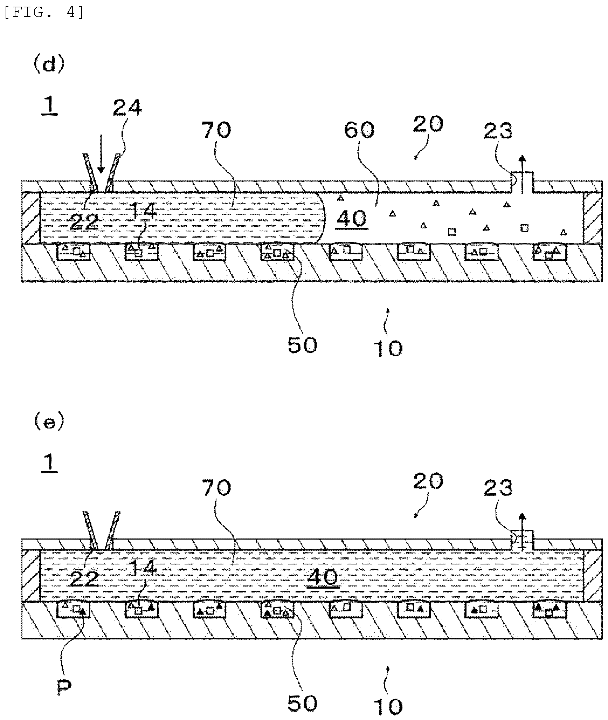

In the present step, the first solvent is caused to flow into the flow path 40 to retain the first solvent in the chamber. Hereinafter, the steps after the first inflow step will be described with reference to Figs. 3 and 4.
As illustrated in Fig. 3(a), a tip 24 of a pipette is inserted into the solvent inlet 22 of the cover 20, and the dextran aqueous solution 50 as the first solvent is caused to flow into the flow path 40 in advance. As a result, the inside of the chamber 14 and the flow path 40 are filled with the dextran aqueous solution 50. The dextran aqueous solution 50 overflowing from the flow path 40 flows out from the solvent outlet 23 of the cover 20.

(5) Second Inflow Step

In the present step, the second solvent 60 is caused to flow into the flow path 40 to bring the first solvent 50 retained in the chamber 14 into contact with the second solvent 60. As a result, the biological substance contained in the second solvent 60 is moved to the first solvent 50 in the chamber 14. In addition, in the present step, when the first solvent 50 remains in the flow path 40, the first solvent is pushed out of the flow path 40. The concentration of the dextran aqueous solution 50 in the chamber 14 hardly changes due to contact with the polyethylene glycol aqueous solution 60, and the concentration in a first solvent introduction step is maintained. Here, the "contact" includes mixing two aqueous solutions, leaving the mixture at a constant temperature for a constant time (incubation), maintaining a stirred state, and the like. A flow rate when the second solvent 60 flows into the flow path 40 can be appropriately set, and can be, for example, 0.1 $\mu$l/sec to 10 $\mu$l/sec. The time (contact time) for bringing the second solvent 60 into contact with the first solvent 50 can be appropriately set according to the properties of these solvents and biological substances, and the like, but is usually 1 minute or longer, preferably 5 minutes or longer, and more preferably 10 minutes or longer. When the contact time is 10 minutes or longer, the biological substance in the second solvent 60 is likely to be sufficiently enriched in the first solvent 50 in the chamber 14. An upper limit of the contact time is not particularly limited, but is usually 120 minutes or shorter, and more preferably 60 minutes or shorter. When the contact time is 60 minutes or shorter, the biological substance enriched in the first solvent 50 in the chamber 14 hardly diffuses into the second solvent 60.
Describing the present step according to the present embodiment, as illustrated in Fig. 3(b), the polyethylene glycol aqueous solution 60 as a second solvent is caused to flow into the flow path 40 from the solvent inlet 22 of the cover 20. As a result, the dextran aqueous solution 50 remaining in the flow path 40 is extruded to the outside from the solvent outlet 23. Since the dextran and the polyethylene glycol have a concentration exceeding the critical concentration, the dextran aqueous solution 50 and the polyethylene glycol aqueous solution 60 are phase-separated from each other. As a result, a dextran layer having a large specific gravity is formed in a lower layer (in the chamber 14), and a polyethylene glycol layer having a small specific gravity is formed in an upper layer (in the flow path 40). As described above, in the method of the present embodiment, the dextran aqueous solution 50 can be retained in the plurality of chambers 14 by a simple operation of introducing the dextran aqueous solution 50 into the flow path 40 as compared with a method using a conventional pipette.
The polyethylene glycol aqueous solution 60 contains the enzyme E and the substrate S as biological substances. Both the enzyme E and the substrate S have a higher distribution coefficient to the dextran aqueous solution 50 than the polyethylene glycol aqueous solution 60. Therefore, as illustrated in Fig. 3(c), when the polyethylene glycol aqueous solution 60 is brought into contact with the dextran aqueous solution 50 retained in the chamber 14, the enzyme E and the substrate S are moved to the dextran aqueous solution 50 in the polyethylene glycol aqueous solution 60 and enriched in the chamber 14. Since the biological substance enriched in the dextran aqueous solution 50 in the chamber 14 is hardly diffused into the polyethylene glycol aqueous solution 60 in the flow path 40 due to a difference in distribution property, the biological substance is retained in a state of being enriched in the dextran aqueous solution 50. Since the dextran aqueous solution 50 and the polyethylene glycol aqueous solution 60 are sufficiently in contact with each other in the chamber 14 and the flow path 40, the biological substance is enriched in the dextran aqueous solution 50 in the chamber 14 at a high concentration. In Fig. 3, the enzyme E and the substrate S as biological substances are mixed with the polyethylene glycol aqueous solution 60, and both are simultaneously introduced into the flow path 40, but the present invention is not limited thereto. For example, a polyethylene glycol aqueous solution mixed only with the enzyme E may be first introduced into the flow path 40 and moved into the dextran aqueous solution 50 in the chamber 14, and then, a polyethylene glycol aqueous solution mixed only with the substrate S may be introduced into the flow path 40 and moved into the chamber 14 to react with the enzyme E. Similarly, a detection reagent may be mixed with the biological substance in a polyethylene glycol aqueous solution and introduced into the flow path 40, or may be mixed with a polyethylene glycol aqueous solution different from the biological substance and introduced into the flow path 40 separately from the biological substance.

(6) Sealing Solvent Inflow Step

In the step, the sealing solvent 70 flows into the flow path 40 to push out the second solvent 60 remaining in the flow path 40 from the flow path 40. Examples of the sealing solvent 70 include hydrophobic solvents such as silicone oil, perfluorocarbon oil, perfluoropolyether oil, a halogen-based solvent, a saturated hydrocarbon solvent, an unsaturated hydrocarbon solvent, and an aromatic hydrocarbon solvent. The sealing solvent 70 preferably has higher hydrophobicity than the first solvent 50 and the second solvent 60. In addition, in order to prevent diffusion of the biological substance, the sealing solvent 70 has a lower distribution property of the biological substance than the dextran aqueous solution 50.

Describing the present step in accordance with the present embodiment, as illustrated in Fig. 4(d), the sealing solvent 70 is caused to flow into the flow path 40 from the solvent inlet 22 of the cover 20. As a result, the polyethylene glycol aqueous solution 60 remaining in the flow path 40 is extruded to the outside from the solvent outlet 23. As a result, as illustrated in Fig. 4(e), the inside of the flow path 40 is filled with the sealing solvent 70, the biological substances (the enzyme E and the substrate S) are retained in the dextran aqueous solution 50 in the chamber 14, and the enzyme-substrate reaction proceeds to produce a product P. That is, the inside of the solvent accommodated in the chamber 14 serves as a biological reaction reactor. As described above, as the inside of the flow path 40 is sealed with the sealing solvent 70, it is possible to prevent the biological substance from diffusing from the chamber 14 into the flow path 40 to maintain an enriched state, and it is also possible to prevent evaporation of the solvent. In this manner, the biological substance is enriched in the dextran aqueous solution 50 in the chamber 14, and the reaction by the biological substance proceeds. The present step of sealing the space 40 with the sealing solvent 70 is optional, and the sealing solvent 70 may be unnecessary in a case where the biological substance has a property of easily moving to the dextran aqueous solution 50. In addition, the type of the sealing solvent is optional, or sealing may be performed with air or the like instead of the sealing solvent.

(7) Modification 1

In the above embodiment, the enzyme E and the substrate S as biological substances are mixed with the polyethylene glycol aqueous solution 60 as the second solvent and enriched in the chamber 14, but the method of the present invention is not limited thereto, and the biological substance may be mixed with the dextran aqueous solution 50 as the first solvent. In this case, the flow cell preparation step and the solvent preparation step are the same as those in the above embodiment, but the mixing step is different from that in the above embodiment in that the biological substance is mixed with the dextran aqueous solution 50. The order of the subsequent steps is the same as that in the above embodiment, but in the first inflow step, the dextran aqueous solution 50 containing the biological substance is retained in the chamber 14. In the second inflow step, the flow path 40 located above the dextran aqueous solution 50 in the chamber 14 is filled with the polyethylene glycol aqueous solution 60. Therefore, the biological substance contained in the dextran aqueous solution 50 is less likely to move to the polyethylene glycol aqueous solution 60, and is retained in the dextran aqueous solution 50. In this manner, the biological substance can be enriched and retained in the chamber 14. Furthermore, an embodiment in which the biological substance is mixed in both the first solvent and the second solvent is also possible.

(8) Modification 2

In the above embodiment, the dextran aqueous solution 50 as the first solvent is caused to flow in the first inflow step, and then, the polyethylene glycol aqueous solution 60 as the second solvent is caused to flow in the second inflow step, but the present invention is not limited thereto, and may be performed in the reverse order.

That is, in the present modification, the polyethylene glycol aqueous solution 60 as the second solvent is caused to flow in the first inflow step, and the dextran aqueous solution 50 as the first solvent is caused to flow in the subsequent second inflow step. Even in this order, it is possible to retain the first solvent in the chamber 14 and enrich the biological substance therein.

(9) Modification 3

It is preferable to further include a step (tag adding step) of adding a tag that improves the distribution property to the first solvent to the biological substance. Such a tag may include a dextran binding domain of dextran sucrase (EC2.4.1.5). As the dextran sucrase, those derived from the genus *Leuconostoc* or the genus *Streptococcus,* which are lactic acid bacteria, can be used, and for example, those derived from *"Leuconostoc mesenteroides"* are preferably used. When the biological substance is a protein, such a tag can be introduced into the biological substance by genetic recombination or the like. In this case, the tag can be introduced at any position such as the C-terminus or N-terminus of the protein.

Examples of such a tag include nucleic acids such as DNA and RNA, chemically modified molecules obtained by chemically modifying these nucleic acids, nucleic acid-like molecule polymers which are analogues of these nucleic acids and chemically modified molecules, dextran itself, and dextran-like molecule polymers. Examples of the nucleic acid-like molecule polymer include those obtained by modifying any one or a plurality of bases, sugars, and phosphoric acids in natural DNA or RNA. Examples of such a base include those obtained by modifying natural adenine, guanine, cytosine, thymine, and uracil, for example, N-methyladenine, N-benzoyladenine, 2-methylthioadenine, 2-aminoadenine, 7-methylguanine, N-isobutyrylguanine, 5-fluorocytosine, 5-bromocytosine, 5-methylcytosine, 4-N-methylcytosine, 4-N,N-dimethylcytosine, 5-fluorouracil, 5-bromouracil, 5-chlorouracil, 5,6-dihydrouracil, and the like. Examples of such a sugar include those obtained by modifying natural ribose and deoxyribose, for example, 2-fluoro-2-deoxyribose, 2-chloro-2-deoxyribose, 2-O-methylribose, 2-O-methoxyethylribose, and morpholino. Furthermore, examples of the phosphoric acid include those obtained by modifying a natural phosphoric acid group, for example, phosphorothioate, methyl phosphonate, and methoxypropyl phosphonate. Examples of the dextran-like molecule polymer include cationized dextran, carboxymethyl dextran, and diethylaminoethyl dextran. In the modification, even when the distribution property of the biological substance to the first solvent is low, the distribution property of the biological substance to the first solvent can be improved by the method of introducing such a tag.

(10) Modification 4

In the embodiment described above, the biological substance is enriched and analyzed by supplying a certain amount of the second solvent into the flow path 40 of the flow cell 1 after supplying the first solvent, but the present invention is not limited thereto. For example, the biological substance may be enriched and analyzed by continuously supplying the second solvent into the flow path 40 of the flow cell 1 by a continuous flow method. In this case, in consideration of the fact that the first solvent in the chamber 14 flows out and diffuses by the second solvent, a mixed solvent obtained by mixing the first solvent in the second solvent may be supplied as the second solvent. A ratio of the first solvent and the second solvent in the mixed solvent can be appropriately set, and can be set to, for example, 0.1:1 to 1:1 in terms of a volume ratio.

2. Reaction Detection Method

Next, a reaction detection method will be described. A reaction detection method includes a detection step of detecting a reaction of the biological substance retained and/or enriched by the biological substance treatment method. In the above embodiment, since the enzyme E and the substrate S are enriched in the chamber 14 in the biological substance treatment method, the enzyme-substrate reaction is detected in the detection step. A detection method used in the detection step can be appropriately set according to the type of the biological substance, and a detection method using a confocal microscope, a fluorescence microscope, an image sensor, or the like can be used. In particular, in a digital bioassay, a system using an image sensor can be preferably used. As an example of such a system, a light source that irradiates the flow cell 1 with light, an image sensor that receives light emitted by a biological reaction of the flow cell 1, and image analysis software that analyzes an image obtained by the image sensor and evaluates a reaction in each chamber 14 are provided. Then, the biological reaction digital analysis can be performed by counting the number of chambers 14 in which the biological reaction is observed among the plurality of chambers 14 using the image analysis software.

3. Biological Substance Treatment Apparatus

Next, a biological substance treatment apparatus will be described. The biological substance treatment apparatus is an apparatus for implementing the biological substance treatment method described above. Specifically, the flow cell 1, the first solvent (the dextran aqueous solution 50 in the present embodiment), and the second solvent (the polyethylene glycol aqueous solution 60 in the present embodiment) are used. Furthermore, means such as a pipette for realizing the mixing step, the first inflow step, and the second inflow step (corresponding to mixing means, first inflow means, and second inflow means) are provided.

4. Reaction Detection Apparatus

Next, a reaction detection apparatus will be described. The reaction detection apparatus is means (corresponding to detection means) for realizing the detection step in the above reaction detection method. Examples of such an apparatus include the above-described confocal microscope, fluorescence microscope, image sensor, and the like.

5. Biological Substance

Examples of the biological substance include various substances. Examples thereof include nucleic acids such as DNA and RNA, peptides, and proteins. These substances may be naturally derived or artificially synthesized. Specific examples of the biological substance include an enzyme, a substrate, an antibody, an antigen, a polymerase, a receptor, a ribosome, a hormone, and a cytokine. As these biological substances, those derived from various organisms such as viruses, prokaryotes, and eukaryotes can be used.

DNA or RNA has a higher distribution property to the dextran aqueous solution 50 than the polyethylene glycol aqueous solution 60, and thus is easily enriched in the chamber 14. In particular, double-stranded DNA having a double helix has a high distribution property to the dextran aqueous solution 50. The protein has a different distribution property to the dextran aqueous solution 50 depending on its properties. For the biological substance having a low distribution property to the dextran aqueous solution 50, the distribution property to the dextran aqueous solution 50 can be improved by adding the above tag.

The reaction by the biological substance can be detected, for example, by measuring fluorescence generated in the biological reaction. Examples of the fluorescent dye (reporter dye) used for measuring fluorescence for a probe of DNA or RNA include FAM, Cy3 (registered trademark), Cy5 (registered trademark), FITC (fluorescein isothiocyanate), TRITC (tetramethylrhodamine B isothiocyanate), TexasRed (registered trademark), rhodamine, and TAMRA. Only one type of reporter dye may be used, or two or more types thereof may be used in combination. When two or more type of reporter dyes are used, two or more types of biological reactions can be detected in one chamber by emitting fluorescence in different reactions by biological substances.

Furthermore, it is preferable to combine quencher molecules that absorb light with respect to the fluorescence wavelengths of these reporter dyes. In this case, when the reporter dye and the quencher molecule are close to each other, fluorescence of the reporter dye is absorbed by the quencher molecule by fluorescence resonance energy transfer (FRET), but when the reporter dye and the quencher molecule are separated from each other, fluorescence by the reporter dye can be detected. Therefore, a reaction such as cleavage with respect to the probe can be detected with higher sensitivity by combining the reporter dye and the quencher molecule. Examples of such a quencher molecule include BHQ (registered trademark) and TAMRA. The reporter dye and the quencher molecule can be used by being bound to the 5' end and the 3' end of a probe of DNA or RNA, respectively.

The present embodiment can be used for enrichment of various biological substances or detection of reactions, and for example, can be preferably used for detection of pathogenic microorganisms such as influenza viruses. Examples of a method for detecting pathogenic microorganisms include a method described in JP 2022-031760 A. For example, an enzyme present on a surface or inside of a pathogenic microorganism and a substance serving as a substrate thereof are used as biological substances, and a product of an enzyme-substrate reaction is detected by an optical method or the like using the flow cell 1 described above. As such a combination of an enzyme and a substrate, a combination described in Table 1 of JP 2022-031760 A or the like can be suitably employed.

As a detection system using fluorescence, various detection systems can be used, and for example, an enzyme-linked immunosorbent assay (ELISA) described in WO 2016/047068 A and the like can be adopted.

Furthermore, a system for detecting a pathogenic microorganism by targeting RNA derived from a pathogenic microorganism is also effective. Details will be described below.

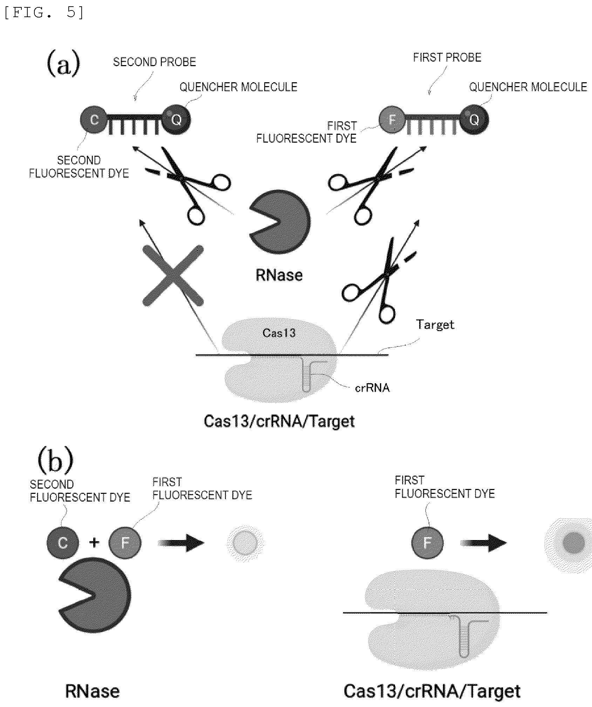

The present system utilizes a reaction that specifically cleaves RNA using Cas13. Specifically, as illustrated in Fig. 5(a), the biological substance includes at least a target RNA ("Target" in the drawing), crRNA for detecting the same, Cas13, and two types of RNA probes (a first probe and a second probe of the drawing). The target RNA is RNA having a sequence specific to the pathogenic microorganism to be detected, and for example, RNA containing ROF1ab gene, N gene, S gene, or the like can be used in SARS-CoV-2 or the like. The crRNA has a sequence complementary to a specific region of the target RNA, and forms a complex with Cas13. The complex binds to the target RNA using the crRNA as a guide RNA to form an activated complex, and cleaves the target RNA.

One of the two RNA probes (the first RNA probe) has a sequence that can be cleaved by the activated complex, and the first fluorescent dye ("F" in the drawing) and the quencher molecule ("Q" in the drawing) are bound. When the RNA of the first RNA probe is cleaved, the first fluorescent dye F and the quencher molecule Q are separated from each other, and fluorescence (first fluorescence) by the fluorescent dye F is generated. On the other hand, the other of the two RNA probes (the second RNA probe) does not have a sequence that can be cleaved by the activated complex, and the second fluorescent dye ("C" in the drawing) and the quencher molecule ("Q" in the drawing) are bound. The second fluorescent dye C emits fluorescence distinguishable from the first fluorescent dye F, and for example, emits fluorescence having a wavelength different from that of the first fluorescent dye F. When the RNA of the second RNA probe is cleaved, the second fluorescent dye C and the quencher molecule Q are separated from each other, and fluorescence (second fluorescence) by the fluorescent dye C is generated.

In the environment of a specimen, a chip, or the like, there is a sequence non-specific ribonuclease that is a ribonuclease (RNase) that cleaves RNA without depending on the sequence, which may contaminate the reaction system. As illustrated on the left side of Fig. 5(b), when RNase is present, both of the two probes (the first probe and the second probe) are cleaved, and both fluoresce of the first fluorescent dye F and the second fluorescent dye C (first fluorescence and second fluorescence). On the other hand, as illustrated on the right side of Fig. 5(b), when there is an activated complex in which the complex of Cas13 and crRNA binds to the target RNA, only the first RNA probe is cleaved and only fluorescence (first fluorescence) by the first fluorescent dye F is emitted. Therefore, when only the first fluorescence is detected in one chamber 14, it can be determined that the signal is a positive signal due to the complex of Cas13. On the other hand, when both the first fluorescence and the second fluorescence are detected in one chamber 14, it can be determined that the signal is a false positive signal due to a sequence non-specific ribonuclease. In the present embodiment, only one type of probe for detecting a false positive is used, but the present invention is not limited thereto, and two or more types of probes may be used. By increasing the number of types of probes for false positive detection, the false positive distinguishability can be enhanced and the detection accuracy of the target can be improved.

A specimen (including a target RNA) containing these biological substances (crRNA, Cas13, and two RNA probes) is mixed with the first solvent or the second solvent and introduced into the flow cell 1. In the examples described below, the second solvent (the polyethylene glycol aqueous solution 60) is mixed and introduced. These biological substances have a higher distribution property to the first solvent (the dextran aqueous solution 50) than the second solvent (the polyethylene glycol aqueous solution 60), and thus are distributed and enriched in the dextran aqueous solution 50 retained in the chamber 14.

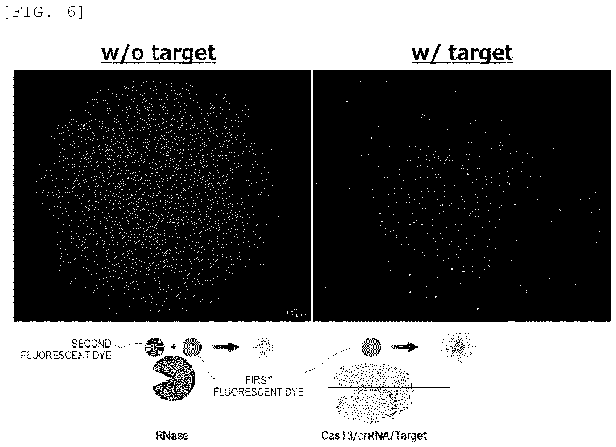

In the chamber 14 where the complex of Cas13 is present, only the first RNA probe is cleaved, and the first fluorescence by the first fluorescent dye F is generated (right side of Fig. 6). Therefore, a positive signal can be measured by measuring the chamber 14 that emits only the first fluorescence (the positive measurement step and the positive measurement means).

On the other hand, when a sequence non-specific ribonuclease is contained in the specimen, it is retained in some of the chambers 14. In the chamber 14 containing the sequence non-specific ribonuclease, both the first RNA probe and the second RNA probe are cleaved, and both the first fluorescence by the first fluorescent dye F and the second fluorescence by the second fluorescent dye C are generated (left side of Fig. 6). Therefore, a false positive signal can be measured by measuring the chamber 14 that emits both the fluorescence (the false positive measurement step and the false positive measurement means).

In this manner, it is possible to improve the detection sensitivity of the target RNA by eliminating the false positive signal.

(1) Modification 6: Capture of Target DNA by DBD-Capturing DNA

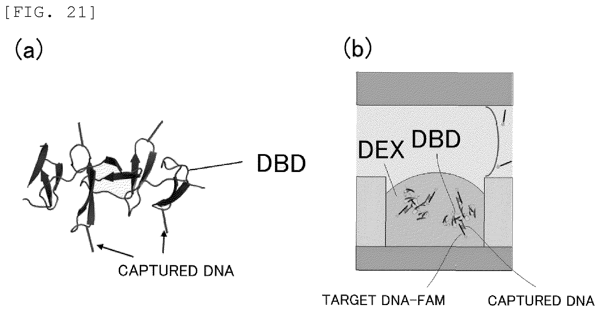

By binding DNA for capture to dextran or a dextran binding domain (DBD), DNA complementary to the DNA for capture can be bound and enriched in the dextran aqueous solution 50. Fig. 21(a) is a schematic view illustrating a state in which a captured DNA is bound to DBD. As illustrated in Fig. 21(b), the DBD-capturing DNA is enriched in the dextran aqueous solution 50. In the drawing, target DNA-FAM to which a fluorescent dye FAM is bound is used as the target DNA. Since the capturing DNA has a sequence complementary to the target DNA and binds to the target DNA, the target DNA-FAM is also enriched in the dextran aqueous solution 50, and the target DNA can be detected by detecting fluorescence by FAM.

Examples

Hereinafter, the present invention will be specifically described with reference to Examples, but these Examples will not limit the object of the present invention. Further, in the following Examples, the expression "%" is on a mass basis (mass percent) unless otherwise particularly specified.

Since an aqueous two-phase system (ATPS) of dextran (DEX) and polyethylene glycol (PEG) can preferentially distribute a nucleic acid polymer such as DNA to a DEX-rich phase, an experiment was performed focusing on this system. First, based on a femtoliter chamber array device (FRAD) developed for a conventional digital bioassay, a system, in which uniformly shaped DEX-rich droplets (hereinafter, the DEX droplets are simply referred to as DEX droplets) were arranged, was developed. In addition, a tag system capable of distributing a protein that is originally difficult to enrich in the DEX-rich phase to DEX droplets was developed. Thereafter, a tagged enzyme such as alkaline phosphatase or Cas13 was enriched on-chip and a sensitive digital bioassay beyond the theoretical LOD by total reaction volume was performed. Details will be described below.

1. Materials and Methods

(1) Chemical Substances

Polyethylene glycol (MW: 35 kDa), dextran from the genus *Leuconostoc* (MW: 450 to 650 kDa), and dextran labeled with TRITC-DEX (tetramethylrhodamine B isothiocyanate (TRITC) (DEX: MW: 500 kDa) were obtained from Merck (Sigma-Aldrich), Germany. Fluorinert-FC40 and FC43 (3M, USA), fluorescein diphosphate (FDP) (AAT Bioquest, USA), SYBR Gold (Thermo Fisher SCIENTIFIC, USA), SURFLON S-386 (AGC Seimi Chemical Co., Ltd., Japan), BSA (bovine serum albumin: NEW ENGLAND Biolabs, USA), and Fomblin Y-LVAC25/6 (Solvay, Belgium), and fluorescein di-β-galactopyranoside (FDG) (Abcam, England) were supplied by their respective suppliers.

(2) Proteins and RNA

A highly active variant ALP (D101 S) of *Escherichia* coli-derived alkaline phosphatase (ALP) was used throughout the experiment and was designated as "ALP" or "EcALP" for simplicity. In order to prepare an ALP tag with a dextran binding domain (DBD) of dextran sucrase of *Leuconostoc mesenteroides* (ALP-DBD) (Suwannarangsee, S.; Moulis, C.; Potocki-Veronese, G.; Monsan, P.; Remaud-Simeon, M.; Chulalaksananukul, W., Search for a dextransucrase minimal motif involved in dextran binding. FEBS Lett 2007, 581(24), 4675-80), it was genetically fused with DBD at the c-terminus of ALP. ALP or ALP-DBD proteins were prepared in the in-vitro TXTL system (PURExpress, NEW ENGLAND Biolabs, USA) reported in the paper below (Ueno, H.; Kato, M.; Minagawa, Y.; Hirose, Y.; Noji, H., Elucidation and control of low and high active populations of alkaline phosphatase molecules for quantitative digital bioassay. Protein Sci 2021, 30(8), 1628-1639). β-galactosidase (Wako, Japan) and anti-β-galactosidase IgY antibodies (Abcam, England) were purchased from their respective suppliers.

In the digital RNA count assay, Cas13a from *Leptotrichia wadei* was used. The Cas13 protein was purified with some modifications according to the report of the following paper (Kellner, M. J.; Koob, J. G.; Gootenberg, J. S.; Abudayyeh, O. O.; Zhang, F., SHERLOCK: nucleic acid detection with CRISPR nucleases. Nat Protoc 2019, 14(10), 2986-3012). Size exclusion chromatography was omitted in the purification step as the DBD obtained from dextran sucrase was able to adhere to a size exclusion chromatography (SEC) column. The target RNA of the digital RNA count assay using Cas13 was designed to be 3,000 nt so as to encode the S gene fragment of SERS-CoV-2 and to be efficiently enriched in the DEX-rich phase. DNA encoding the designed RNA was synthesized by SYNTHGO, Corporation, USA. RNA was prepared from the synthesized DNA using the ScriptMax Thermo T7 Transcription Kit (TOYOBO, Japan) and the NucleoSpin RNA clean-up Kit (Macherey-Nagel). Synthesis of crRNA was performed using a device from Synthgo.

The sequence of each RNA is as follows.

(1) Target RNA

GGGUAACAUCACUAGGUUUCAAACUUUACUUGCUUUACAUAGAAGUUAUUU

GACUCCUGGUGAUUCUUCUUCAGGUUGGACAGCUGGUGCUGCAGCUUAUUAUGUGGG

UUAUCUUCAACCUAGGGAGAACAGCAAGAAGCACGAGAAGUACAAGAUCCGCGAGUA

CUAUCACAAGAUCAUCGGCCGGAAGAACGACAAAGAGAACUUCGCCAAGAUUAUCUA

CGAAGAGAUCCAGAACGUGAACAACAUCAAAGAGCUGAUUGAGAAGAUCCCCGACAU

GUCUGAGCUGAAGAAAAGCCAGGUGUUCUACAAGUACUACCUGGACAAAGAGGAACU

GAACGACAAGAAUAUUAAGUACGCCUUCUGCCACUUCGUGGAAAUCGAGAUGUCCCA

GCUGCUGAAAAACUACGUGUACAAGCGGCUGAGCAACAUCAGCAACGAUAAGAUCAA

GCGGAUCUUCGAGUACCAGAAUCUGAAAAAGCUGAUCGAAAACAAACUGCUGAACAA

GCUGGACACCUACGUGCGGAACUGCGGCAAGUACAACUACUAUCUGCAAGUGGGCGA

GAUCGCCACCUCCGACUUUAUCGCCCGGAACCGGCAGAACGAGGCCUUCCUGAGAAA

CAUCAUCGGCGUGUCCAGCGUGGCCUACUUCAGCCUGAGGAACAUCCUGGAAACCGA

GAACGAGAACGAUAUCACCGGCCGGAUGCGGGGCAAGACCGUGAAGAACAACAAGGG

CGAAGAGAAAUACGUGUCCGGCGAGGUGGACAAGAUCUACAAUGAGAACAAGCAGAA

CGAAGUGAAAGAAAUCUGAAGAUGUUCUACAGCUACGACUUCAACAUGGACAACAA

GAACGAGAUCGAGGACUUCUUCGCCAACAUCGACGAGGCCAUCAGCAGCAUCAGACA

CGGCAUCGUGCACUUCAACCUGGAACUGGAAGGCAAGGACAUCUUCGCCUUCAAGAA

UAUCGCCCCCAGCGAGAUCUCCAAGAAGAUGUUUCAGAACGAAAUCAACGAAAAGAA

GCUGAAGCUGAAAAUCUUCAAGCAGCUGAACAGCGCCAACGUGUUCAACUACUACGA

GAAGGAUGUGAUCAUCAAGUACCUGAAGAAUACCAAGUUCAACUUCGUGAACAAAAA

CAUCCCCUUCGUGCCCAGCUUCACCAAGCUGUACAACAAGAUUGAGGACCUGCGGAA

UACCCUGAAGUUUUUUUGGAGCGUGCCCAAGGACAAAGAAGAGAAGGACGCCCAGAU

CUACCUGCUGAAGAAUAUCUACUACGGCGAGUUCCUGAACAAGUUCGUGAAAAACUC

CAAGGUGUUCUUUAAGAUCACCAAUGAAGUGAUCAAGAUUAACAAGCAGCGGAACCA

GAAAACCGGCCACUACAAGUAUCAGAAGUUCGAGAACAUCGAGAAAACCGUGCCCGU

GGAAUACCUGGCCAUCAUCCAGAGCAGAGAGAUGAUCAACAACCAGGACAAAGAGGA

AAAGAAUACCUACAUCGACUUUAUUCAGCAGAUUUUCCUGAAGGGCUUCAUCGACUA

CCUGAACAAGAACAAUCUGAAGUAUAUCGAGAGCAACAACAACAAUGACAACAACGA

CAUCUUCUCCAAGAUCAAGAUCAAAAAGGAUAACAAAGAGAAGUACGACAAGAUCCU

GAAGAACUAUGAGAAGCACAAUCGGAACAAAGAAAUCCCUCACGAGAUCAAUGAGUU

CGUGCGCGAGAUCAAGCUGGGGAAGAUUCUGAAGUACACCGAGAAUCUGAACAUGUU

UUACCUGAUCCUGAAGCUGCUGAACCACAAAGAGCUGACCAACCUGAAGGGCAGCCU

GGAAAAGUACCAGUCCGCCAACAAAGAAGAAACCUUCAGCGACGAGCUGGAACUGAU

CAACCUGCUGAACCUGGACAACAACAGAGUGACCGAGGACUUCGAGCUGGAAGCCAA

CGAGAUCGGCAAGUUCCUGGACUUCAACGAAAACAAAUCAAGGACCGGAAAGAGCU

GAAAAAGUUCGACACCAACAAGAUCUAUUUCGACGGCGAGAACAUCAUCAAGCACCG

GGCCUUCUACAAUAUCAAGAAAUACGGCAUGCUGAAUCUGCUGGAAAAGAUCGCCGA

UAAGGCCAAGUAUAAGAUCAGCCUGAAAGAACUGAAAGAGUACAGCAACAAGAAGAA

UGAGAUUGAAAAGAACUACACCAUGCAGCAGAACCUGCACCGGAAGUACGCCAGACC

CAAGAAGGACGAAAAGUUCAACGACGAGGACUACAAAGAGUAUGAGAAGGCCAUCGG

CAACAUCCAGAAGUACACCCACCUGAAGAACAAGGUGGAAUUCAAUGAGCUGAACCU

GCUGCAGGGCCUGCUGCUGAAGAUCCUGCACCGGCUCGUGGGCUACACCAGCAUCUG

GGAGCGGGACCUGAGAUUCCGGCUGAAGGGCGAGUUUCCCGAGAACCACUACAUCGA

GGAAAUUUUCAAUUUCGACAACUCCAAGAAUGUGAAGUACAAAAGCGGCCAGAUCGU

GGAAAAGUAUAUCAACUUCUACAAAGAACUGUACAAGGACAAUGUGGAAAAGCGGAG

CAUCUACUCCGACAAGAAAGUGAAGAAACUGAAGCAGGAAAAAAGGACCUGUACAU

CCGGAACUACAUUGCCCACUUCAACUACAUCCCCCACGCCGAGAUUAGCCUGCUGGA

AGUGCUGGAAACCUGCGGAAGCUGCUGUCCUACGACCGGAAGCUGAAGAACGCCAU

CAUGAAGUCCAUCGUGGACAUUCUGAAAGAAUACGGCUUCGUGGCCACCUUCAAGAU

CGGCGCUGACAAGAAGAUCGAAAUCCAGACCCUGGAAUCAGAGAAGAUCGUGCACCU

GAAGAAUCUGAAGAAAAGAAACUGAUGACCGACCGGAACAGCGAGGAACUGUGCGA

ACUCGUGAAAGUCAUGUUCGAGUACAAGGCCCUGGAAUAAGCGGCCGCACUCGAGGC

CCGAAAGGAAGCUGAGUUGGCUGCUGCCACCGCUGAGCAAUAA

(2) crRNA

GAUUUAGACUACCCCAAAAACGAAGGGGACUAAAAC<u>GCAGCACCAGCUGUC</u>

<u>CAACCUGAAGAAG</u>

(* The underline indicates a complementary sequence between the target RNA and the crRNA.)
(3) Femtoliter Chamber Array Device (FRAD)

According to the report of the paper, a FRAD in which a plurality of chambers were arranged was produced by microfabrication by photolithography (the above paper by Ueno, H. et al.). The flow cell was assembled using a FRAD (corresponding to a chamber array device), a top glass with holes at an inlet and an outlet (corresponding to a cover), and a double-sided tape (80 $\mu$m or less) as a spacer. The top glass of the flow cell chamber was precoated with CYTOP 809M to avoid non-specific binding of biomolecules.

(4) Imaging with Microscope

Confocal fluorescence images were acquired using a laser scanning confocal microscope TCS SP8X (Leica Microsystems, Germany) equipped with a white light laser (Leica Microsystems, Germany). The fluorescence signal of TRITC-DEX was acquired using a HyD detector (Leica Microsystems, Germany). Confocal fluorescence images were analyzed using Fiji, which is an image processing package of ImageJ. In addition, epi-fluorescence images were obtained using an epi-fluorescence microscope (ECLIPSE Ti2, Nikon, or Olympus IX83, Olympus Corporation) equipped with an sCMOS camera (Zyla sCMOS or Andor neo, Andor Technology) and an LED light source (X-Cite TURBO or X-cite XYLIS, Excelitas technologies). The epi-fluorescence images were acquired using Fiji (Image Processing Package of ImageJ) and custom written macros.

(5) Formation of Uniform DEX Reactor by FRAD

A DEX solution at a 5.5% (w/w) indicated concentration containing 0.1% (w/w) TRITC-DEX was injected into the flow cell chamber. Thereafter, PEG at the indicated concentration (5.0% (w/w)) was injected, and an excess amount of DEX solution was caused to flow from the flow path. The DEX solution remained within each micron-sized cavity on the FRAD, and uniformly shaped DEX droplets were formed. For a DNA enrichment experiment, a DNA solution in 5% (w/w) PEG containing 12.5 ng/$\mu$l of $\lambda$-DNA stained with x1 SYBR gold was injected into the flow cell.

(6) Digital Bioassay of ALP-DBD by DEX Droplet System

Prior to the assay, a blocking solution (Tween20 0.2% (w/v)) was injected into the flow cell chamber in order to prevent non-specific binding of ALP molecules to the flow cell surface. Thereafter, an ALP buffer in which 4% (w/w) DEX and 0.1% (w/w) TRITC-DEX were mixed was injected, and the ALP buffer was injected into the flow cell chamber pretreated with the blocking solution. Thereafter, ALP or ALP-DBD was introduced into an ALP buffer containing 4% (w/w) PEG. Then, after 10 minutes of incubation, the PEG solution was flushed with FC40 oil, and then, Fomblin oil was injected to prevent evaporation.

(7) Digital RNA Measurement of Cas13a by DEX Droplet System

In order to distinguish the RNA cleavage reaction by LwaCas13a from non-specific ribonucleases, self-quenched probes were prepared. LwaCas13a preferentially cleaved AU, UU, CU, and GU dinucleotide motifs, but showed low activity with respect to the remaining motifs (Gootenberg, J. S.; Abudayyeh, O. O.; Kellner, M. J.; Joung, J.; Collins, J. J.; Zhang, F., Multiplexed and portable nucleic acid detection platform with Cas13, Cas12a, and Csm6. Science 2018, 360(6387), 439-444). FAM-AU-BHQ1 (5'-6FAM-taAUgc-BHQ1-3'), which is a self-quenching probe for cleavage of Cas13, and Cy5-AC-BHQ3 (5'-Cy5-taACgc-BHQ3-3'), which is a self-quenching probe for non-specific ribonuclease were both obtained from Fasmac, Japan. The upper case of the probe name represents ribonucleotide (that is, RNA), and the lower case represents deoxyribonucleotide (that is, DNA).

Details of the procedure of digital RNA counting by Cas13 using DEX droplets are as follows. First, a blocking solution containing 0.5 mg/ml of BSA, 0.06% (w/w) S-386, 500 nM FAM-AU-BHQ1, and 500 nM Cy5-AC-BHQ3 was injected into the flow cell chamber in Cas13 buffer (20 mM HEPES-NaOH, pH 6.8, 60 mM NaCl, 6 mM MgCl$_2$). Subsequently, DEX mix (5.5% (w/w) DEX, 0.1% (w/w) TRITC-DEX in Cas13 buffer) was injected into the flow cell, and PEG mix (5% (w/w) PEG, 0.06% (w/w) S-386 in Cas13 buffer) flowed. After 10 minutes of incubation, the following components were injected into the PEG mix: 45 nM LwaCas13a-DBD, 22.5 nM crRNA, 3 $\mu$M FAM-AU-BHQ1, 10 $\mu$M Cy5-AU-BHQ3, 2U/ml RNase inhibitor, and 0.06% (w/w) S-386.

In addition, an encapsulated oil (1:1 mixture of FC43 and Fomblin Y-LVAC 25/6) was introduced. After 30 minutes of incubation at room temperature, fluorescence images were acquired with an epi-fluorescence microscope. After injection of the blocking solution, a reaction mix (labeled amounts of target RNA, 45 nM LwaCas13a-DBD, 22.5 nM crRNA, 3 $\mu$M FAM-AU-BHQ1, 10 $\mu$M Cy5-AU-BHQ3, 2 U/ml RNase inhibitor, and 0.06% S-386 in Cas13 buffer) was injected to perform a digital bioassay without DEX droplets. Finally, sealing oil (1:1 mixture of FC43 and Fomblin Y-LVAC 25/6) was injected into the flow cell chamber.

2. Results

(1) Femtoliter DEX Droplet Array System

(Experimental Example 1)

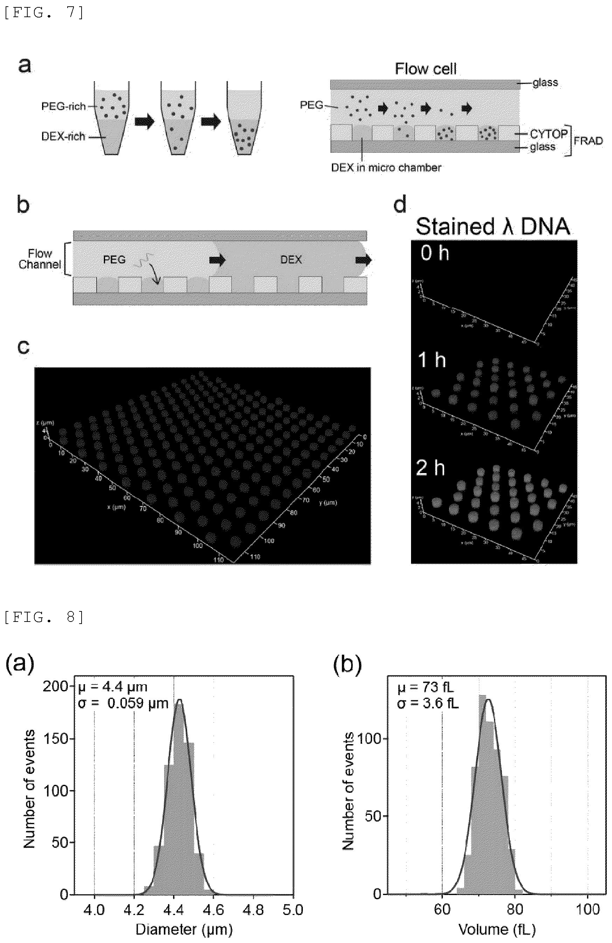

An overview and results of an experiment using the DEX droplet array system is illustrated in Fig. 7. (a) of the drawing is a conceptual view that enriches biomolecules with the conventionally known DEX-rich phase in a normal test tube (left) and DEX droplets in DEX/PEG ATPS on a femtoliter chamber array device (FRAD) (right). (b) of the drawing illustrates a schematic view of a formation process of the DEX droplets on the FRAD. (c) of the drawing is a confocal image of the DEX droplets formed on the FRAD. When the DEX solution was introduced into the flow cell chamber, the DEX droplets were stained by adding 0.1% (w/w) TRITC-DEX in 5.5% (w/w) DEX. (d) of the drawing is a time-lapse image obtained by capturing a state in which the fluorescent-stained A-DNA in the DEX droplets is enriched on the chip by frame every hour.

For preparation of femtoliter-sized uniform DEX droplets, a FRAD which can display 1,000,000 micron-sized reactors (diameter: 4.4 $\mu$m, height: 3.2 $\mu$m) was employed. As illustrated in Fig. 7(b), the preparation procedure can be described as follows. First, a DEX solution was injected into a flow cell chamber in which a FRAD system was placed at the bottom, and a reactor was filled with the DEX solution. Thereafter, the PEG solution was introduced into the flow cell chamber, and the excess DEX solution was washed. As a result, as illustrated in Fig. 7(c), DEX-rich droplets were formed in each reactor of the FRAD under the PEG solution. When the concentrations of DEX and PEG were 4.0 to 5.5 wt%, formation of DEX droplets was confirmed.

Fig. 8 is a view illustrating a diameter and a volume of a DEX reactor formed in the femtoliter chamber array device. (a) of the drawing illustrates a diameter of the DEX reactor obtained by analyzing the confocal microscope image of the TRITC-DEX fluorescence of Fig. 7, and (b) of the drawing illustrates a volume thereof. $\mu$ and $\sigma$ on the upper left in the drawing represent an average and a standard deviation obtained by Gaussian fitting. It can be seen that the DEX droplets formed in the reactor exhibited high volume uniformity of 73 $\pm$ 3.6 fL (cv = 4.9%). As described above, uniform DEX droplets having a femtoliter volume were produced by a simple procedure using the FRAD system.

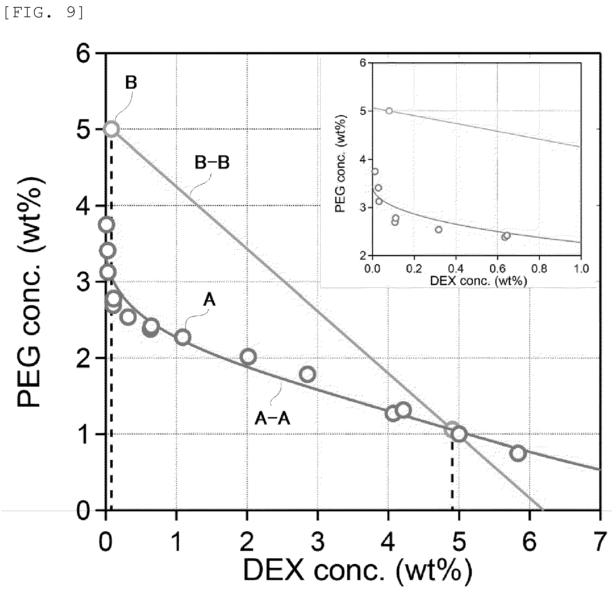

Fig. 9 illustrates a binomial curve and an estimated tie line. The circle (A) indicates a dilution point at which DEX/PEG ATPS becomes a miscible solution, and is determined by a titration method. Line (A-A) indicates the results of fitting with the formula. $[PEG] = C_1 exp(C_2[DEX]^{0.5} + C_3[DEX]^3)$

The circle (B) indicates the concentration of 5.5% (w/w) DEX containing 0.03% (w/w) TRITC-DEX and 5.0% (w/w) PEG mixed at a ratio of 1:125, and indicates the intersection with the binomial curve at the concentration of the DEX-rich phase (4.9% (w/w)) after mixing. Line (B-B) indicates the tie line connecting two circles (B).

The initial concentrations of DEX and PEG were 5.5 wt% for DEX and 5.0 wt% for PEG, considering that the initial concentrations were sufficient to cause phase separation and to maintain a moderate viscosity for reproducible handling of the solution. Considering a ratio of the total reaction volume of FRAD and the flow path (1:100 or more in the present Example), when the flow path solution is replaced with a PEG solution, the final concentration of DEX in the flow path chamber should approximate 0.05 wt% or less. In fact, it has been confirmed that phase separation occurs when 5.5 wt% DEX and 5.0 wt% PEG are mixed at 1:125 in a conventional tube (Fig. 9). Under this condition, the DEX concentration of the DEX-rich phase was 4.9 wt%. Thus, DEX-rich droplets can be conveniently formed on the FRAD after injecting 100 times the amount of PEG solution into the flow path.

It was verified whether arrayed DEX droplets have the ability to enrich DNA molecules. According to previous reports on ATPS of DEX/PEG systems (Nakatani, N.; Sakuta, H.; Hayashi, M.; Tanaka, S.; Takiguchi, K.; Tsumoto, K.; Yoshikawa, K., Specific Spatial Localization of Actin and DNA in a Water/Water Microdroplet: Self-Emergence of a Cell-Like Structure. Chembiochem 2018, 19(13), 1370-1374), double-stranded DNA molecules of kbp in length are effectively enriched in DEX-rich phase. In order to test this phenomenon on the device, a DNA molecule with 48 kbp in length was introduced into the flow cell with 5.5 wt% PEG. As illustrated in Fig. 7(d), the DNA molecules were highly enriched over time to show an efficient enrichment similar to the DEX-rich phase prepared in vitro.

(2) Digital Bioassay of Alkaline Phosphatase by DEX Droplet System (Experimental Example 2)

It was examined whether the DEX droplet array system can be used for a standard digital bioassay using *Escherichia* coli-derived alkaline phosphatase (ALP) and EcALP. DNA and RNA molecules are preferentially distributed to the DEX-rich phase, while some globular proteins are inefficiently enriched to the DEX-rich phase. In order to efficiently enrich for EcALP, DBD from *Leuconostoc mesenteroides* dextran sucrase25 and EcALP were genetically fused.

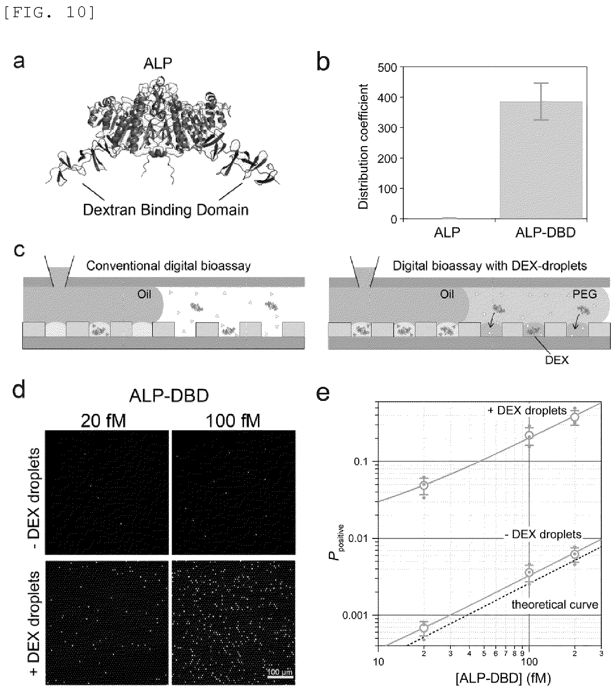

Fig. 10 is a view illustrating results of a digital bioassay of EcALP by the DEX droplet system. (a) of the drawing illustrates ALP derived from *Escherichia coli* having DBD at the C-terminus (ALP-DBD). The structure was modeled with alphafold. (b) of the drawing illustrates distribution coefficients (DC) of ALP and ALP-DBD in DEX/PEG ATPS. Error bars in the drawing represent s.d. (n = 3). (c) of the drawing is a schematic view of a conventional digital bioassay (left) and a digital bioassay using DEX droplets (right).

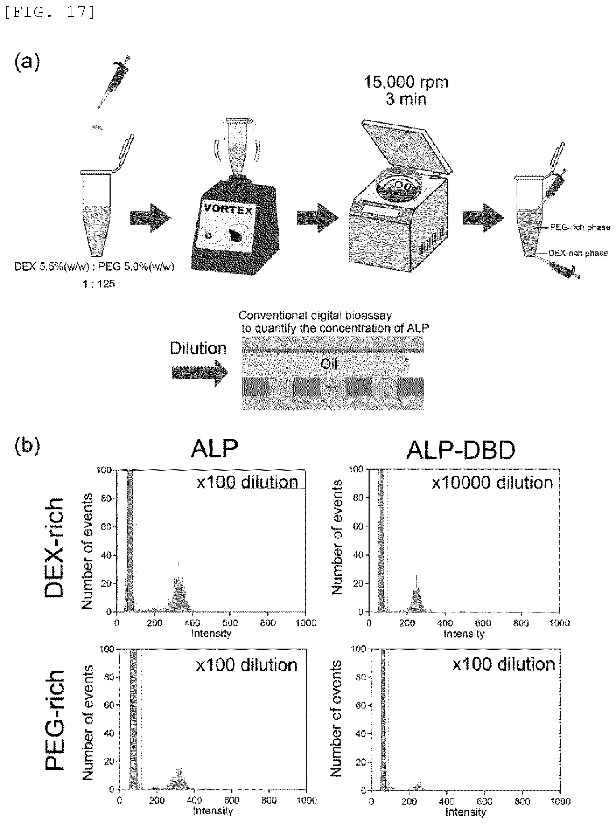

DBD is a relatively small protein (14 kDa) and has a high affinity for dextran (Kd: 2.79 x $10^{-9}$ M) (Wu, S. C.; Wang, C.; Chin, J.; Wong, S. L., A bio-coupling approach using a dextran-binding domain to immobilize an engineered streptavidin to Sephadex for easy preparation of affinity matrix. Sci Rep 2019, 9(1), 3359). The distribution coefficient (DC) of DBD-tagged EcALP (ALP-DBD) to the DEX-rich phase formed in a normal test tube was measured (Fig. 17).

Fig. 17 is a view illustrating distribution coefficients of ALP and ALP-DBD to ATPS for DEX/PEG. Fig. 17(a) is a schematic view of an experimental protocol for estimating a distribution coefficient. Fig. 17(b) is a histogram of fluorescence intensity by fluorescein that is a product obtained by hydrolyzing FDP by ALP. The upper left and lower left are histograms of ALP in the collected DEX-rich phase and PEG-rich phase. The upper right and the upper left are histograms of ALP-DBD in the DEX-rich phase and the PEG-rich phase diluted 10,000 times and 100 times, respectively.

ALP-DBD was placed in a tube in which 5.5 wt% DEX/5wt% PEG was mixed at 1:125, and centrifuged to separate a PEG-rich phase at the top and a DEX-rich phase at the bottom. Thereafter, an aliquot of each phase was collected, and the ALP concentration was measured. In order to measure an EcALP concentration, a standard digital bioassay of ALP was used. In principle, DC is determined as $C_{DEX}/C_{PEG}$, where $C_{DEX}$ and $C_{PEG}$ represent the concentrations of ALP-DBD in the DEX- and PEG-rich phases, respectively.

(3) Quantification of Distribution Coefficient of ALP-DBD

In order to separate the DEX-rich phase at the bottom and the PEG-rich phase at the top, 0.04% (w/w) DEX, 0.001% TRITC-DEX, 5% (w/w) PEG, 0.5 nM ALP or ALP-DBD, a reaction buffer for ALP (ALP buffer; 1 M diethanolamine, pH 9.25, 1 mM $MgCl_2$, 0.02% (w/v), Tween20) were appropriately mixed, and centrifuged at 15,000 rpm for 3 minutes. Thereafter, an aliquot was sampled from each phase. After dilution with a reaction mix for ALP (10 $\mu$M Alexa647, 1 mM FDP, 1 M diethanolamine, pH 9.25, 1 mM $MgCl_2$, 0.02% (w/v), Tween20), an ALP concentration in the sample was quantified by a digital bioassay. The results are illustrated in Fig. 10(b).

As shown in Fig. 10(b), DC of intact EcALP was 1.9, whereas that of ALP-DBD was 385. EcALP is slightly enriched in the DEX-rich phase, but when a DBD tag is attached, it is enriched at a ratio of about 203 times. This means that the enzyme tagged with DBD is efficiently distributed to the DEX-rich phase. It should be noted that since EcALP is a homodimer enzyme, ALP-DBD needs to include two DBDs.

A digital bioassay with ALP-DBD and DEX droplet array system was performed. ALP-DBD molecules were introduced into a flow cell chamber of a FRAD with a reactor mixed in a 5.5 wt% PEG solution and filled with a 5 wt% DEX solution. After 10 minutes of incubation, an oil solution was introduced to seal the DEX droplets (Fig. 10(c)).

Fig. 10(d) is fluorescence images of the digital bioassay without DEX droplets (top) and with DEX droplets (bottom) in 20 fM, 100 fM ALP-DBD. For comparison, an assay without DEX/PEG solution (upper part) was also performed. A digital bioassay using DEX droplets displayed a clearly higher number of positive reactants at each concentration of ALP-DBD.

Fig. 10(e) illustrates a frequency of positive reactors ($P_{positive}$) vs. [ALP-DBD]. The circles on the straight line of "+DEX droplets" indicate $P_{positive}$ of the digital bioassay using DEX droplets, and the circles on the straight line of "-DEX droplets" indicate $P_{positive}$ of the assay without DEX droplets, respectively. The circles indicate an average value of $P_{positive}$ (n = 3), and the error bars indicate s.d. The broken line indicates the theoretical value of $P_{positive}$ from the total reactor capacity. The straight line of "+DEX droplets" and the red line of "-DEX droplets" indicate fittings for determining the coefficients of proportionality to [ALP], respectively. $1.9 \times 10^{-3}$ [/fM] in the assay using DEX droplets, and $3.2 \times 10^{-5}$ [/fM] in the assay not using DEX droplets. The enrichment factor determined as the ratio of these factors was 59.

The drawing illustrates the probability of a positive reaction reactor with and without ATPS of DEX/PEG. The data points not containing DEX droplets sharply drop near the theoretical line estimated from the ALP-DBD concentration, but the data points containing DEX droplets show a clearly high value. The data points were fitted with equations consisting of concentration-dependent and independent terms. The enrichment factor was determined as the ratio of concentration factor. As a result, the enrichment factor of the DEX droplets was 59 times compared with the assay using no DEX droplets. As described above, it was confirmed that the DEX droplet system efficiently enriches the DBD-labeled protein without applying an external device or an external force.

(4) Digital Bioassay for RNA Detection by DEX Droplet System with Cas13

Enrichment power of DEX droplets was studied in a digital RNA detection assay, which is a more practical form. Cas13 is a ribonuclease induced by crRNA, and exhibits trans-cleaved RNase activity (referred to as securing activity) by recognizing a target RNA via a complementary sequence on the crRNA previously loaded on the Cas13 protein. Since the secondary activity of Cas13 is sufficiently high and can be easily detected using a self-quenching fluorescent reporter RNA, a highly sensitive RNA detection method using Cas13 protein was developed (Kellner, M. J.; Koob, J. G.; Gootenberg, J. S.; Abudayyeh, O. O.; Zhang, F., SHERLOCK: nucleic acid detection with CRISPR nucleases. Nat Protoc 2019, 14(10), 2986-3012, Liu, T. Y.; Knott, G. J.; Smock, D. C. J.; Desmarais, J. J.; Son, S.; Bhuiya, A.; Jakhanwal, S.; Prywes, N.; Agrawal, S.; Derby, M. D. D.; Switz, N. A.; Armstrong, M.; Harris, A. R.; Charles, E. J.; Thornton, B. W.; Fozouni, P.; Shu, J.; Stephens, S. I.; Kumar, G. R.; Zhao, C. Y.; Mok, A.; Iavarone, A. T.; Escajeda, A. M.; McIntosh, R.; Kim, S. E.; Dugan, E. J.; Pollard, K. S.; Tan, M. X.; Ott, M.; Fletcher, D. A.; Lareau, L. F.; Hsu, P. D.; Savage, D. F.; Doudna, J. A.; Consortium, I. T., Accelerated RNA detection using tandem CRISPR nucleases. Nat Chem Biol 2021, 17(9), 982-988, Mahas, A.; Wang, Q. C.; Marsic, T.; Mahfouz, M. M., A Novel Miniature CRISPR- Cas13 System for SARS-CoV-2 Diagnostics. Acs Synthetic Biology 2021, 10(10), 2541-2551).

Cas13-based digital bioassays for RNA detection have also been reported (Tian, T.; Shu, B.; Jiang, Y.; Ye, M.; Liu, L.; Guo, Z.; Han, Z.; Wang, Z.; Zhou, X., An Ultralocalized Cas13a Assay Enables Universal and Nucleic Acid Amplification-Free Single-Molecule RNA Diagnostics. ACS Nano 2021, 15(1), 1167-1178, Shinoda, H.; Taguchi, Y.; Nakagawa, R.; Makino, A.; Okazaki, S.; Nakano, M.; Muramoto, Y.; Takahashi, C.; Takahashi, I.; Ando, J.; Noda, T.; Nureki, O.; Nishimasu, H.; Watanabe, R., Amplification-free RNA detection with CRISPR-Cas13. Commun Biol 2021, 4(1), 476).

For example, in order to detect SARS- COVID-19, a Cas13-based high-speed digital bioassay was developed based on a FRAD system displaying a 3 fL capacity reactor. This system allows rapid detection of SARS-COVID-19 RNA within several minutes. However, the total volume of the reactor is proportionally small, and as a result, LOD is limited to 10 fM or higher unless a pull-down enrichment procedure using a magnetic bead and an external magnetic system is employed (Shinoda, H.; Iida, T.; Makino, A.; Yoshimura, M.; Ishikawa, J.; Ando, J.; Murai, K.; Sugiyama, K.; Muramoto, Y.; Nakano, M.; Kiga, K.; Cui, L.; Nureki, O.; Takeuchi, H.; Noda, T.; Nishimasu, H.; Watanabe, R., Automated amplification-free digital RNA detection platform for rapid and sensitive SARS-CoV-2 diagnosis. Commun Biol 2022, 5(1), 473). As described above, the trade-off between the detection time and the detection sensitivity is one of the technical challenges of the digital bioassay using Cas13. In the present embodiment, the detection sensitivity is improved by performing on-chip enrichment without using an external device in digital detection of SARS-COVID-19 RNA using a DEX droplet array.

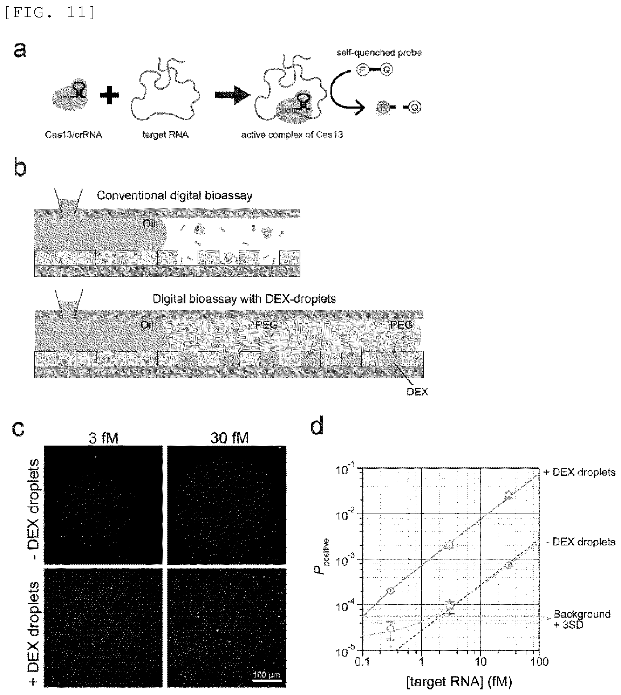

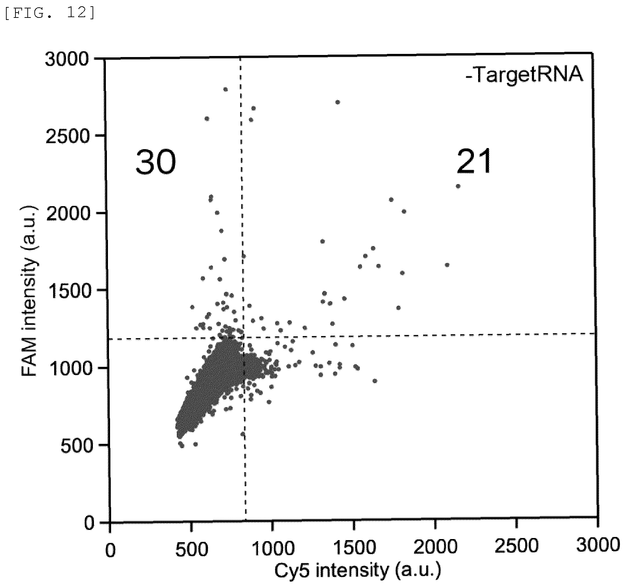

Fig. 11 illustrates a digital RNA counting experiment by Cas13 using the DEX droplet system. (a) of the drawing is a schematic view of RNA detection by a Cas13 system, and illustrates a detection method used for a digital counting method of an RNA molecule (3,000 nt) encoding a SARS-CoV-2 S gene using Cas13. As a fluorescent reporter substrate, a synthetic oligonucleotide having an AU sequence at the cleavage site of Cas13 was used. In the preliminary experiment, there were two technical problems. One is that even in the absence of the target RNA, a false positive signal is observed at a frequency of 0.015% when calculated from Fig. 12 described below. The false positive reaction does not result from fluorescent impurities in the solution or on the device because it increases fluorescence over time. This is considered to be due to contamination of non-specific ribonuclease from the sample or environment, and when another type of reporter oligonucleotide having no recognition sequence for Cas13 was added, fluorescence was emitted regardless of the presence or absence of the recognition sequence for Cas13. The reporter with the recognition site for Cas13 is designed to emit green fluorescence, and the reporter without the recognition site is designed to emit red fluorescence. As described above, the dual reporter system facilitates distinguishing between the concomitant activity of Cas13 as a green fluorescence signal and the concomitant activity of non-specific ribonucleases that generate both fluorescence signals.

Fig. 11(b) is a schematic view of a conventional digital bioassay (upper part) and a digital bioassay using the DEX droplet system of the present embodiment (lower part). In the conventional method, a Cas13/crRNA/target RNA complex and a self-quenching probe are mixed and injected into a flow cell chamber. On the other hand, in an assay using DEX droplets, a target RNA is enriched in a DEX reactor, and then, Cas13/crRNA and a self-quenched probe are introduced into a flow cell chamber.

Fig. 12 illustrates experimental results of a dual reporter system that suppresses a false positive signal. The reporters with and without the recognition site for Cas13 was designed to emit FAM and Cy5 fluoresce, respectively. The fluorescence intensities of FAM and Cy5 obtained from a Cas13 based digital bioassay in the absence of target RNA were two-dimensionally plotted. A reactor emitting FAM fluorescence of a threshold value (horizontal broken line) or more is a false positive signal. Under conditions of low concentrations of the target RNA, these false positive reactors have a relatively strong effect on $P_{positive}$. Among them, those having Cy5 fluorescence intensity equal to or higher than the threshold value can be excluded from the analysis as false positive signals. From the drawing, it was found that in the dual reporter system, the false positive signal was suppressed to about 40% with respect to the positive signal. The origin of the remaining false positives is thought to vary.

Another technical problem found in the preliminary experiments was that the frequency of positive reaction reactors was not as high as expected from the distribution coefficient of RNA into the DEX-rich phase. It was assumed that the target RNA molecule is once enriched in the DEX solution, but a portion exposed to the outside of the target RNA bound to the Cas13 protein is digested by the activated Cas13, and the target RNA-Cas13 complex is released from the DEX-droplet. Based on this assumption, Cas13a tagging was performed using DBD repeated 2 times. As a result, the frequency of positive reactions was significantly increased, and the above hypothesis was supported.

Fig. 11(c) is a fluorescence image obtained with the target RNA molecules of 3 fM and 30 fM with or without DEX droplets. The upper part illustrates the results of the assay using no DEX droplets, and the lower part illustrates the results of the assay using DEX droplets. Clearly, the assay with DEX droplets detected more fluorescent reactants than the assay without ATPS.

Fig. 11(d) illustrates the probability of positive reactors ($P_{positive}$) against the target RNA concentration experimentally determined in the assay with DEX droplets (+DEX droplets) or without DEX droplets (-DEX droplets). The black line indicates the theoretical $P_{positive}$ estimated from the total reactor capacity. The circles indicate an average value of $P_{positive}$ (n = 3), and the error bars indicate s.d. The solid line is a fitting curve for determining a percentage factor with respect to [target RNA]. The percentage factor is $7.5 \times 10^{-4}$ [/fM] for the assay with DEX droplets and $2.4 \times 10^{-5}$ for the assay without DEX droplets. From the ratio of these factors, the enrichment factor was estimated to be 31. The broken line indicates the limit of detection (LOD) and the concentration corresponds to the mean value of false positive signals observed without target RNA + 3s.d.

From the drawing, the data points showed sufficient linearity relative to the target concentration in each assay, with the exception of data points without 0.3 fM DEX droplets, regardless of the presence or absence of DEX droplets. The data point without 0.3 fM DEX droplets is a point where $P_{positive}$ is relatively strongly affected by the false positive reaction reactor. Based on linear fitting of the data points, the enrichment factor determined by ATPS was 31. As a result, the detection limit (LOD) was 0.089 fM, which was lower than the theoretical LOD of 0.47 fM estimated from the total reactor capacity. As described above, ATPS can improve the LOD beyond theoretical limits due to its enrichment power.

Conventionally, flow-focus microfluidic systems have been reported as a method for preparing monodisperse DEX droplets (Mastiani, M.; Seo, S.; Jimenez, S. M.; Petrozzi, N.; Kim, M. M., Flow regime mapping of aqueous two-phase system droplets in flow-focusing geometries. Colloid Surface A 2017, 531, 111-120, Zhang, Q. Q.; Chen, J. Q.; Gai, H. W., High-throughput-generating water-in-water droplet for monodisperse biocompatible particle synthesis. J Mater Sci 2019, 54(24), 14905-14913, Zhou, C.; Zhu, P.; Han, X.; Shi, R.; Tian, Y.; Wang, L., Microfluidic generation of ATPS droplets by transient double emulsion technique. Lab Chip 2021, 21(14), 2684-2690). However, in order to avoid natural fusion of the droplets, the droplets often solidified, and the fluidity or internal hydration of the droplets, which are important for the enrichment of biomolecules within the DEX droplets, were impaired. In addition, since the size of the DEX droplets produced by the microfluidic system is large, a digital bioassay utilizing enzyme activity has been practically difficult.

In the present example, a FRAD was used to establish a new method of preparing regularly-shaped DEX droplets with a femtoliter volume. The femtoliter DEX droplets developed in Examples were placed on micron-sized cavities that were arranged without solidification to enter the chamber. Therefore, it was confirmed that the droplet retained the original properties of the DEX-rich phase droplet.

In Examples, on-chip enrichment of DNA, RNA, and protein, followed by a digital bioassay was performed utilizing the function of enriching biomolecules from PEG-rich media of the DEX droplets. When DBD was added to the protein under this condition, a 30-fold or more enrichment of the ALP molecule and the Cas13/RNA complex was achieved. As a result, Cas13 achieved mole detection at or below the femto level by counting digital RNA without using an off-chip enrichment method or a pull-down method using an external device such as a magnetic system or an electrode. The characteristics of the digital bioassay using such DEX droplets enable a novel design strategy of a mobile system equipped with a high-sensitivity digital bioassay, and there is great expectation for realization of a portable diagnostic system in the home medical field.

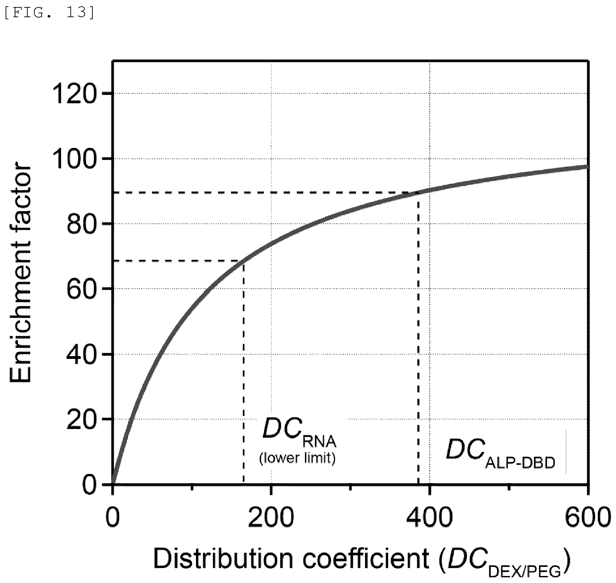

Fig. 13 illustrates a model of an enrichment factor in a digital assay using DEX droplets. Here, a relationship between the enrichment factor and the distribution coefficient in the tube in a digital bioassay using DEX droplets was modeled. The distribution coefficient obtained in the tube is given by the following equation.

[Math. 1]

$$DC_{DEX/PEG} = \frac{C_{DEX}}{C_{PEG}},$$

(Where $C_{DEX}$ and $C_{PEG}$ represent the concentrations of the targets in the DEX-rich phase and the PEG-rich phase. The number of molecules in the solution is preserved.)

Since the number of molecules in the solution is preserved, it is as follows.

[Math. 2]

$$C_{PEG} \cdot V_{PEG} + C_{DEX} \cdot V_{DEX} = C_0 \cdot V_0,$$

(Where $C_0$ represents the initial concentration of the target. $V_0$, $V_{DEX}$, and $V_{PEG}$ represent the total volume, the volume of DEX in the microchamber, and the volume of the PEG-rich phase in the flow path, respectively (Figs. 7(a) and 7(b)).)

The enrichment factor can be expressed as $C_{DEX}/C_0$.

[Math. 3]

$$enrichement = \frac{C_{DEX}}{C_0} = \frac{\frac{V_0}{V_{PEG}}}{\frac{C_{PEG}}{C_{DEX}} + \frac{V_{DEX}}{V_{PEG}}} = \frac{\frac{V_0}{V_{PEG}}}{\frac{1}{P_{DEX/PEG}} + \frac{V_{DEX}}{V_{PEG}}}$$

Here, a volume ratio of PEG and DEX is defined as follows.

[Math. 4]

$$R_{DEX/PEG} = \frac{V_{DEX}}{V_{PEG}}.$$

$R_{DEX/PEG}$ was 1/125 in the digital bioassay using DEX droplets, indicating that $V_{PEG}$ is much larger than $V_{DEX}$. Therefore, this can be expressed as $V_0 \approx V_{PEG}$.

In conclusion, the enrichment factor can be expressed as follows.

[Math. 5]

$$enrichement = \frac{1}{\left(DC_{DEX/PEG}\right)^{-1} + R_{DEX/PEG}}.$$

The distribution coefficient of ALP-DBD in the tube was estimated to be 385, and the distribution coefficient of RNA was estimated to be at least 164. The enrichment factor of ALP-DBD estimated from the model was estimated to be 90, and the enrichment factor in the digital Cas13 measurement was estimated to be at least 68. Both the results were higher than the experimental values. From the ratio of the reactor volume (= total amount of DEX droplets) to the flow cell volume, that is, about 1/100, the upper limit on the degree of enrichment is about 100 (Fig. 13).

In the present system, two major determinants of the enrichment factor are the volume ratio of the reactor volume and the flow cell volume and the distribution coefficient of the DBD-tagged protein. A larger flow cell volume results in a higher enrichment rate, but the enrichment takes a longer time.

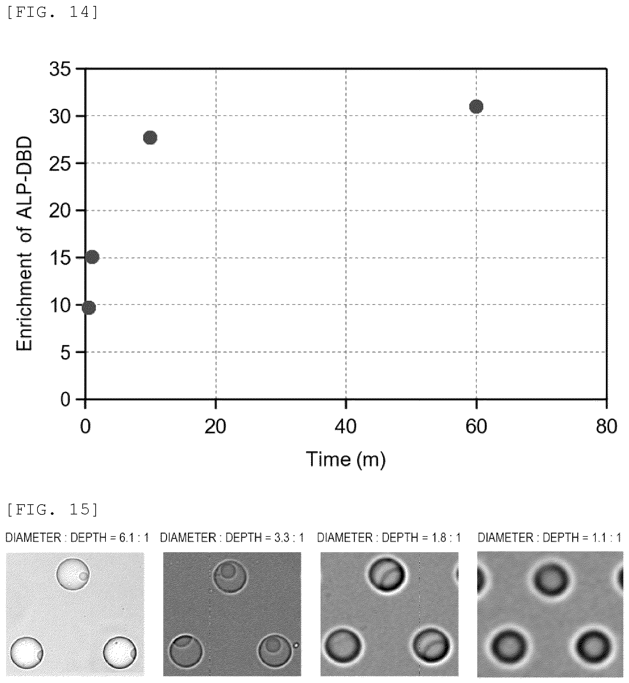

Fig. 14 is a view illustrating an incubation period dependency of an enrichment of ALP-DBD. Time dependence of enrichment of ALP-DBD in a digital bioassay using DEX droplets is shown. The incubation period shown on the horizontal axis represents the time from injection of PEG containing 400 fM ALP-DBD to washing away with FC40. The enrichment factor shown on the vertical axis was determined based on the probability ratio of the positive reaction reactor.

From the drawing, it can be seen that most of the enzyme molecules were enriched within 10 minutes in the present embodiment. When the flow cell chamber is doubled in thickness, the enrichment process requires more than four times the time. Continuous dosing of the buffer can thus avoid an exponential extension of the enrichment process. More importantly, an increase in the distribution coefficient of the DBD protein is also a beneficial strategy. In addition to introducing more DBD into the enzyme, it is considered effective to develop DBD with higher affinity.

In addition, a dual reporter system was developed to reduce the false positive signal of a sensitive digital RNA count assay using the Cas13 protein. False positive signals that can be identified in a dual reporter system can be derived from the non-specific ribonuclease contaminated from the sample or environment. Since ribonucleases are ubiquitous in biological samples, the present method may be useful for digital RNA count analysis of various types of clinical samples.

**4.** Experiment in which Ratio of Diameter/Depth of Chamber is Changed

A chamber in which a ratio of the diameter and the depth was changed was prepared, and formation of a dextran reactor in the chamber was attempted. The diameter of the chamber was fixed to 3 μm, and etching was performed while changing the depth to produce a plurality of types of FRADs having different chamber depths. The height of the flow path 40 through which the solvent flowed was 80 μm. The dextran aqueous solution was caused to flow at a rate of about 1 μl/sec. The results are illustrated in Fig. 15. As the ratio of the diameter to the depth increased, the dextran partially remained without filling the entire chamber.

5. Antibody Enrichment Experiment Using Tag That Improves Distribution Ratio to DEX.

A tag (DBD tag) that increases the distribution ratio to dextran was attached to the antibody, and the antibody was enriched to dextran formed in the chamber in a polyethylene glycol phase. Next, a polyethylene glycol phase containing an antigen against an antibody (here, β-galactosidase: βgal) and a fluorescent substrate was flowed. Thereafter, FC40 and Fomblin oil were caused to flow to perform sealing. After 10 minutes, the number of bright spots was counted by digital measurement using a fluorescence microscope. As a result, the number of bright spots was about 108 times compared with a case where dextran and the antibody were not present. This result indicates that the antigen can be enriched by the antibody enriched in dextran, and most of the antigens in the solution could be enriched in the reactor. Hereinafter, details of the experiment and the results will be described.

In order to demonstrate the extensibility of on-chip enrichment methods on untagged proteins, a DEX droplet system including DBD-tagged IgY (Ab-DBD) was developed. As a model of the target protein, β-galactosidase, which is a homotetramer enzyme, was employed. IgY molecules against β-galactosidase were labeled with DBD to produce Ab-DBD and introduced into DEX droplets. β-Galactosidase molecules added to the PEG solution were injected into a flow cell chamber together with a fluorescent substrate. After 10 minutes of incubation, the DEX droplets were sealed with oil and a digital bioassay of β-galactosidase was performed. A digital bioassay without DEX droplets and/or Ab-DBD were also performed for comparison.

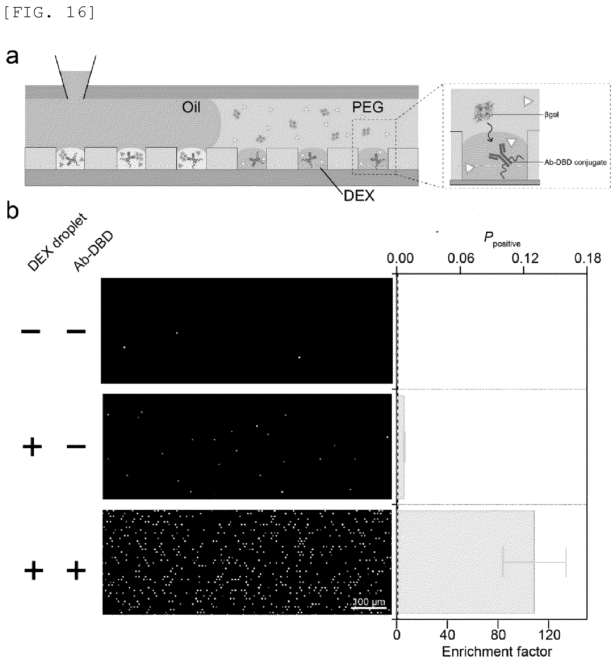

Fig. 16 is a view illustrating an on-chip enrichment of the non-tag enzyme by DEX droplets using Ab-DBD. (a) of the drawing illustrates a schematic view of the on-chip enrichment of a β-galactosidase enzyme. In order to efficiently capture the enzyme, antibody molecules against β-galactosidase are loaded onto DEX droplets prior to enzyme enrichment. (b) of the drawing is a fluorescence image of the digital bioassays without DEX droplets and Ab-DBD (-,-), with DEX droplets and without Ab-DBD (+,-), and with DEX droplets and Ab-DBD (+,+). The right bar graph shows the number of positive reaction reactors (%), $P_{positive}$, and the enrichment factor for the (-, -) condition. The (+,-) was 6.0, and the (+,+) was 108.

From this result, the digital bioassay (DEX droplet was present, Ab-DBD was absent: middle part) showed a high positive reaction number with respect to the conventional bioassay (DEX droplet was absent, Ab-DBD was absent: upper part). On the other hand, the number of positive reactions was remarkably high in the case of the presence of DEX droplets and Ab-DBD (lower part). As a result, the enrichment factors of the DEX droplet and Ab-DBD were 108 times, and a value significantly close to 125 times as the theoretical maximum value was shown. The enrichment factor is even higher than the experimental results of DBD-ALP and DBD-Cas13. This high enrichment power is considered to be because the number of DBD is 4 or more in the complex of β-galactosidase and Ab-DBD, and the distribution coefficient of the complex in the DEX-rich phase is higher than that of DBD-ALP or DBD-Cas13 in which the number of DBD is 1.

6. Experiment in which Biological Substance is Placed Only in DEX Using DEX as First Solvent and PEG as Second Solvent

In the experiment described above, a biological substance such as ALP was mixed with PEG as the second solvent. An experiment was performed to confirm whether the assay was possible even when the biological substance was mixed with the first solvent (DEX).

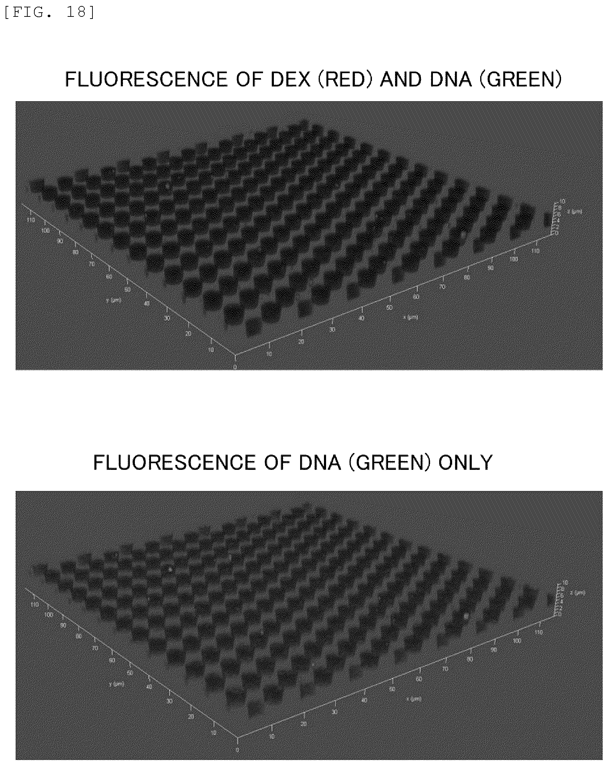

(1) Mixing was performed so that the final concentration of DEX (550 kDa or less) was 5%, TRITC-DEX was 0.1%, and LambdaDNA was 5 nM. TRITC-DEX was prepared by purchasing one sold by MERCK (product code 52194) and diluting it with ultrapure water (MQ). LambdaDNA was purchased from Nippon Gene and prepared by dilution with MQ.
(2) 15 μL of the mixed solution was caused to flow through the flow cell.
(3) 80 μL of 5.5% PEG (35 kDa or less) containing SybrGold for staining DNA with fluorescence was caused to flow into the flow cell, and DEX in the flow path on the chamber was swept away. SybrGold purchased from ThermoFisherSCIENTIFIC (S11494) was mixed into PEG solution to x1 concentration.
(4) Fluorescence of SybrGold and TRITC-DEX was observed using a HyD detector (Leica Microsystems, Germany) using a laser scanning confocal microscope TCS SP8 X (Leica Microsystems, Germany) equipped with a white light laser (Leica Microsystems, Germany). The results are illustrated in Fig. 18.

The upper part of the drawing illustrates fluorescence of DNA fluorescently stained by TRITC-DEX and SybrGold, and the lower part illustrates fluorescence of only DNA. As illustrated in the drawing, even when PEG was flowed after DEX to which the biological substance (DNA) was added was flowed, DNA was retained in DEX formed in the chamber, and the chamber in which fluorescence was generated could be confirmed.

7. Experiment in which DEX and PEG are Reversed (Experiments Showing that PEG is Retained in Chamber Previously Using PEG as First Solvent and Could Be Subsequently Washed with DEX as Second Solvent)

In the experiment described in Fig. 3 and the like, DEX was used as the first solvent and PEG was used as the second solvent, and DEX was first introduced into the chamber and then washed with PEG. An experiment was performed to confirm whether the assay was possible even when the first solvent was replaced with PEG and the second solvent was replaced with DEX.

(1) DEX and PEG were mixed so as to be Final 10%/10%, respectively, and centrifuged under the conditions of 13,000 rpm and 5 min.

(2) The phase-separated PEG phase (upper phase) and DEX phase (lower phase) were separately recovered by inserting an injection needle to the upper side and the lower side of a container (Eppendorf tube) and sucking them with a syringe.

(3) FITC-PEG was mixed with the PEG phase recovered as described above so as to be 0.2%, and 15 $\mu$L of FITC-PEG was caused to flow into the flow path of the flow cell. FITC-PEG is a purchase from CreativePEGWorks and was prepared by dilution with MQ.

(4) 80 $\mu$L of the DEX phase recovered as described above was flowed into the flow path, and PEG in the flow path on the chamber was swept away.

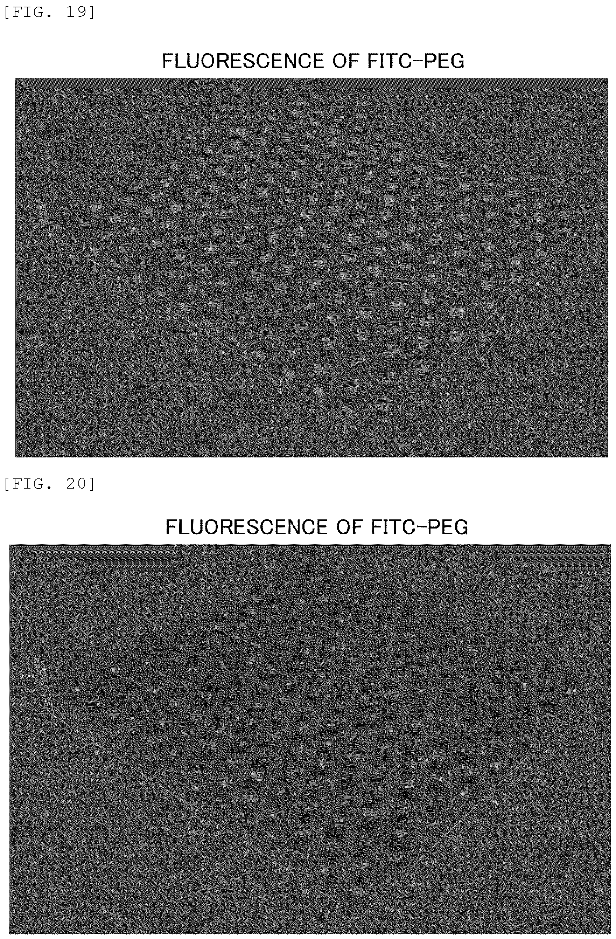

(5) Fluorescence of FITC-PEG was observed using a HyD detector (Leica Microsystems, Germany) using a laser scanning confocal microscope TCS SP8 X (Leica Microsystems, Germany) equipped with a white light laser (Leica Microsystems, Germany). The results are illustrated in Fig. 19.

As illustrated in the drawing, even when PEG as a first solvent was introduced into the flow path of the flow cell and then washed with DEX as a second solvent, a chamber in which fluorescence derived from FITC-PEG was generated could be confirmed. This indicates that PEG is retained in the chamber.

8. Results with PEG as First Solvent and Phosphoric Acid as Second Solvent

In the experiment described above, PEG was used as the first solvent and DEX was used as the second solvent, but an experiment for confirming whether an assay can be performed with other solvents was performed.

(1) Mixing was performed so that the final concentration of PEG (35 kDa or less) was 10% and the final concentration of FITC-PEG was 0.1%.

(2) 15 $\mu$L of the mixed solution was caused to flow through the flow path of the flow cell.

(3) 80 $\mu$L of 1 M phosphoric acid ($K_3PO_4$) was flowed, and PEG in the flow path on the chamber is swept away.

(4) Fluorescence of FITC-PEG was imaged using a HyD detector (Leica Microsystems, Germany) using a laser scanning confocal microscope TCS SP8 X (Leica Microsystems, Germany) equipped with a white light laser (Leica Microsystems, Germany). The results are illustrated in Fig. 20.

As illustrated in the drawing, even when phosphoric acid was used as the second solvent, a chamber in which fluorescence derived from FITC-PEG was generated could be confirmed. As described above, formation of a microreactor using phase separation has been confirmed without being limited to the combination of DEX and PEG.

Reference Signs List

1    Flow cell
10    Base
11    Chamber array device
12    Bottom surface
13    Side surface
14    Chamber
20    Cover
21    Cover body
22    Solvent inlet
23    Solvent outlet
24    Chip
30    Spacer
40    Flow path
50    First solvent (dextran aqueous solution)
60    Second solvent (polyethylene glycol aqueous solution)
70    Sealing solvent (oil)
E    Enzyme

S    Substrate
P    Product

**Claims**

1. A biological substance treatment method for retaining and/or enriching a biological substance, the biological substance treatment method comprising:

   a flow cell preparation step of preparing a flow cell including a chamber array device including a plurality of chambers each having a recess opened at one end, one of which is 1 nanoliter or less, and a cover defining a flow path communicating with the opening of the chamber and provided in common for the plurality of chambers;
   a solvent preparation step of preparing a first solvent and a second solvent that are aqueous to each other and separate into phases when left to stand at room temperature, the first solvent having a property of separating to the chamber side when phase separation occurs in a region surrounded by the chamber and the flow path of the flow cell, and the first solvent and the second solvent having a property that the biological substance is preferentially distributed to the first solvent rather than the second solvent;
   a mixing step of mixing the biological substance with the first solvent and/or the second solvent;
   a first inflow step of causing one solvent of the first solvent and the second solvent to flow into the flow path to fill the chamber and the flow path with the solvent; and
   a second inflow step of causing the first solvent, the other solvent of the first solvent and the second solvent, and the other of the second solvent to flow into the flow path to phase-separate the first solvent into the chamber side and the second solvent into the flow path side, retaining, in the chamber, the biological substance contained in the first solvent in the chamber without diffusing into the second solvent, and/or moving the biological substance contained in the second solvent into the first solvent in the chamber to enrich the biological substance.

2. The biological substance treatment method according to claim 1, wherein the first solvent and the second solvent are selected from combinations indicated by Nos. 1 to 12 in Table 1.

[Table 1]

| No. | First solvent | Second solvent |
|---|---|---|
| 1 | Dextran aqueous solution | Polypropylene glycol aqueous solution |
| 2 | Polyethylene glycol aqueous solution | Polypropylene glycol aqueous solution |
| 3 | Dextran sulfate aqueous solution | Polyethylene glycol aqueous solution |
| 4 | Dextran sulfate aqueous solution | Carboxymethylcellulose aqueous solution |
| 5 | Polyethylene glycol aqueous solution | Phosphoric acid aqueous solution |
| 6 | Polyethylene glycol aqueous solution | Ficoll aqueous solution |
| 7 | Polyethylene glycol aqueous solution | Gelatin aqueous solution |
| 8 | polyU aqueous solution | Spermine aqueous solution |
| 9 | RGG domain + NaCl aqueous solution | Water |
| 10 | DDX4 N-terminal domain + NaCl aqueous solution | Water |
| 11 | Prion-like domain + NaCl aqueous solution | Water |
| 12 | Dextran aqueous solution | Aqueous solution containing copolymer of polyethylene glycol and polypropylene glycol |

3. The biological substance treatment method according to claim 2, wherein the first solvent is a dextran aqueous solution, and the second solvent is a polyethylene glycol aqueous solution.

4. The biological substance treatment method according to claim 1, further comprising a tag adding step of adding a tag that improves a distribution property to the first solvent to the biological substance.

5. The biological substance treatment method according to claim 4, wherein the tag is selected from the group consisting of a dextran binding domain derived from a lactic acid bacterium *"Leuconostoc mesenteroides",* dextran, a dextran-like molecule polymer, DNA, RNA, a chemically modified molecule thereof, and a nucleic acid-like molecule polymer.

6. The biological substance treatment method according to claim 1, further comprising, after the second inflow step, a sealing solvent inflow step of causing a sealing solvent to flow into the flow path to push out the solvent remaining in the flow path from the flow path.

7. A reaction detection method comprising a detection step of detecting a reaction of the biological substance retained and/or enriched by the biological substance treatment method according to claim 1.

8. The reaction detection method according to claim 7, wherein the biological substance includes at least:

   a target RNA;
   crRNA having a sequence complementary to a specific region of the target RNA;
   Cas13 that forms a complex with the crRNA, the Cas13 cleaving the target RNA when the crRNA binds to the target RNA as a guide RNA to form an activated complex;
   a first RNA probe having a sequence capable of being cleaved by the activated complex and binding a first fluorescent dye, the first RNA probe emitting a first fluorescence by being cleaved by the activated complex or a sequence non-specific ribonuclease; and
   a second RNA probe that does not have a sequence to be cleaved by the activated complex, the second RNA probe binding a second fluorescent dye that emits fluorescence distinguishable from the first fluorescent dye and emitting a second fluorescence by being cleaved by the sequence non-specific ribonuclease, and
   the detection step includes:

      a positive measurement step of measuring a chamber emitting only the first fluorescence as a positive signal; and
      a false positive measurement step of measuring a chamber emitting both the first fluorescence and the second fluorescence as false positive signals by the sequence non-specific ribonuclease.

9. A biological substance treatment apparatus for retaining and/or enriching a biological substance, the biological substance treatment apparatus comprising:

   a flow cell including a chamber array device including a plurality of chambers each having a recess opened at one end, one of which is 1 nanoliter or less, and a flow path communicating with the opening of the chamber and provided in common for the plurality of chambers;
   a first solvent and a second solvent that are aqueous to each other and separate into phases when left to stand at room temperature, the first solvent having a property of separating to the chamber side when phase separation occurs in a region surrounded by the chamber and the flow path of the flow cell, and the first solvent and the second solvent having a property that the biological substance is preferentially distributed to the first solvent rather than the second solvent;
   means for mixing the biological substance with the first solvent and/or the second solvent;
   first inflow means for causing one solvent of the first solvent and the second solvent to flow into the flow path to fill the chamber and the flow path with the solvent; and
   second inflow means for causing the other solvent of the first solvent and the second solvent to flow into the flow path to phase-separate the first solvent into the chamber side and the second solvent into the flow path side, bringing the first solvent and the second solvent into contact with each other, retaining, in the chamber, the biological substance contained in the first solvent in the chamber without diffusing into the second solvent, and/or moving the biological substance contained in the second solvent into the first solvent in the chamber to enrich the biological substance.

10. The biological substance treatment apparatus according to claim 9, wherein the first solvent and the second solvent are selected from combinations indicated by Nos. 1 to 12 in Table 2.

[Table 2]

| No. | First solvent | Second solvent |
|---|---|---|
| 1 | Dextran aqueous solution | Polypropylene glycol aqueous solution |
| 2 | Polyethylene glycol aqueous solution | Polypropylene glycol aqueous solution |

(continued)

| No. | First solvent | Second solvent |
|---|---|---|
| 3 | Dextran sulfate aqueous solution | Polyethylene glycol aqueous solution |
| 4 | Dextran sulfate aqueous solution | Carboxymethylcellulose aqueous solution |
| 5 | Polyethylene glycol aqueous solution | Phosphoric acid aqueous solution |
| 6 | Polyethylene glycol aqueous solution | Ficoll aqueous solution |
| 7 | Polyethylene glycol aqueous solution | Gelatin aqueous solution |
| 8 | polyU aqueous solution | Spermine aqueous solution |
| 9 | RGG domain + NaCl aqueous solution | Water |
| 10 | DDX4 N-terminal domain + NaCl aqueous solution | Water |
| 11 | Prion-like domain + NaCl aqueous solution | Water |
| 12 | Dextran aqueous solution | Aqueous solution containing copolymer of polyethylene glycol and polypropylene glycol |

11. The biological substance treatment apparatus according to claim 10, wherein the first solvent is a dextran aqueous solution, and the second solvent is a polyethylene glycol aqueous solution.

12. The biological substance treatment method according to claim 9, wherein the biological substance includes a tag that improves a distribution property to the first solvent.

13. The biological substance treatment apparatus according to claim 12, wherein the tag is selected from the group consisting of a dextran binding domain derived from a lactic acid bacterium *"Leuconostoc mesenteroides"*, dextran, a dextran-like molecule polymer, DNA, RNA, a chemically modified molecule thereof, and a nucleic acid-like molecule polymer.

14. The biological substance treatment apparatus according to claim 9, further comprising a sealing solvent.

15. A reaction detection apparatus further comprising detection means for detecting a reaction of the biological substance retained and/or enriched by the biological substance treatment apparatus according to claim 9.

16. The reaction detection apparatus according to claim 15, wherein the biological substance includes at least:

   a target RNA;
   crRNA having a sequence complementary to a specific region of the target RNA;
   Cas13 that forms a complex with the crRNA, the Cas13 cleaving the target RNA when the crRNA binds to the target RNA as a guide RNA to form an activated complex;
   a first RNA probe having a sequence capable of being cleaved by the activated complex and binding a first fluorescent dye, the first RNA probe emitting a first fluorescence by being cleaved by the activated complex or a sequence non-specific ribonuclease; and
   a second RNA probe that does not have a sequence to be cleaved by the activated complex, the second RNA probe binding a second fluorescent dye that emits fluorescence distinguishable from the first fluorescent dye and emitting a second fluorescence by being cleaved by the sequence non-specific ribonuclease, and
   the detection means includes:

   positive measurement means for measuring a chamber emitting only the first fluorescence as a positive signal; and
   false positive measurement means for measuring a chamber emitting both the first fluorescence and the second fluorescence as false positive signals by the sequence non-specific ribonuclease.

17. A biological substance treatment apparatus for retaining and/or enriching a biological substance using a flow cell including a chamber array device including a plurality of chambers each having a recess opened at one end, one of which is 1 nanoliter or less, and a flow path communicating with the opening of the chamber and provided in common for the plurality of chambers, the biological substance treatment apparatus comprising:

a first solvent and a second solvent that are aqueous to each other and separate into phases when left to stand at room temperature, the first solvent having a property of separating to the chamber side when phase separation occurs in a region surrounded by the chamber and the flow path of the flow cell, and the first solvent and the second solvent having a property that the biological substance is preferentially distributed to the first solvent rather than the second solvent;

means for mixing the biological substance with the first solvent and/or the second solvent;

first inflow means for causing one of the first solvent and the second solvent to flow into the flow path to fill the chamber and the flow path with the solvent; and

second inflow means for causing the other of the first solvent and the second solvent to flow into the flow path to phase-separate the first solvent into the chamber side and the second solvent into the flow path side, retaining, in the chamber, the biological substance contained in the first solvent in the chamber without diffusing into the second solvent, and/or moving the biological substance contained in the second solvent into the first solvent in the chamber to enrich the biological substance.

[FIG. 1]

[FIG. 2]

EP 4 606 907 A1

[FIG. 3]

(a)

(b)

(c)

[FIG. 4]

(d)

(e)

[FIG. 5]

[FIG. 6]

**w/o target**      **w/ target**

EP 4 606 907 A1

[FIG. 7]

[FIG. 8]

39

[FIG. 9]

[FIG. 10]

[FIG. 11]

[FIG. 12]

[FIG. 13]

[FIG. 14]

[FIG. 15]

DIAMETER : DEPTH = 6.1 : 1    DIAMETER : DEPTH = 3.3 : 1    DIAMETER : DEPTH = 1.8 : 1    DIAMETER : DEPTH = 1.1 : 1

[FIG. 16]

[FIG. 17]

[FIG. 18]

## FLUORESCENCE OF DEX (RED) AND DNA (GREEN)

## FLUORESCENCE OF DNA (GREEN) ONLY

[FIG. 19]

## FLUORESCENCE OF FITC-PEG

[FIG. 20]

## FLUORESCENCE OF FITC-PEG

[FIG. 21]

(a)

DBD

CAPTURED DNA

(b)

DEX DBD

TARGET DNA-FAM          CAPTURED DNA

## INTERNATIONAL SEARCH REPORT

| International application No. |
|---|
| **PCT/JP2023/037489** |

| A. | CLASSIFICATION OF SUBJECT MATTER |
|---|---|

*C12Q 1/6806*(2018.01)i; *C12M 1/00*(2006.01)i; *C12M 1/34*(2006.01)i; *C12N 9/22*(2006.01)i; *C12N 15/10*(2006.01)i; *C12Q 1/34*(2006.01)i; *C12Q 1/6876*(2018.01)i; *G01N 1/10*(2006.01)i; *G01N 1/40*(2006.01)i
FI: C12Q1/6806 Z ZNA; C12M1/00 A; C12M1/34 B; C12N9/22; C12N15/10 100Z; C12Q1/34; C12Q1/6876 Z; G01N1/10 F; G01N1/40

According to International Patent Classification (IPC) or to both national classification and IPC

| B. | FIELDS SEARCHED |
|---|---|

Minimum documentation searched (classification system followed by classification symbols)

C12Q1/6806; C12M1/00; C12M1/34; C12N9/22; C12N15/10; C12Q1/34; C12Q1/6876; G01N1/10; G01N1/40

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | TABATA, K. V. et al. Antibody-free digital influenza virus counting based on neuraminidase activity. Scientific Reports. 2019, vol. 9, 1067, <https://doi.org/10.1038/s41598-018-37994-6> abstract, materials and methods, sections "FRAD", "DIViC procedure in the FRAD", fig. 1, etc. | 1-3, 6, 7, 9-11, 14, 15, 17 |
| A | abstract, materials and methods, sections "FRAD", "DIViC procedure in the FRAD", fig. 1, etc. | 4, 5, 8, 12, 13, 16 |
| Y | SIMON, A. B. et al. Aqueous two-phase systems enable multiplexing of homogeneous immunoassays. TECHNOLOGY. 2014, vol. 2, no. 2, pp. 176-184, <doi:10.1142/S2339547814500150> abstract, p. 176, right column, line 12 to p. 177, left column, line 7, fig. 1b, etc. | 1-3, 6, 7, 9-11, 14, 15, 17 |
| A | abstract, p. 176, right column, line 12 to p. 177, left column, line 7, fig. 1b, etc. | 4, 5, 8, 12, 13, 16 |

☑ Further documents are listed in the continuation of Box C.    ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **21 December 2023** | **09 January 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

International application No.

**PCT/JP2023/037489**

| C. | DOCUMENTS CONSIDERED TO BE RELEVANT | |
|---|---|---|
| Category* | Citation of document, with indication, where appropriate, of the relevant passages | Relevant to claim No. |
| Y | NAKATANI, N. et al. Specific Spatial Localization of Actin and DNA in a Water/Water Microdroplet: Self-Emergence of a Cell-Like Structure. ChemBioChem. 2018, vol. 19, pp. 1370-1374, <DOI:10.1002/cbic.201800066> abstract, etc. | 1-3, 6, 7, 9-11, 14, 15, 17 |
| A | abstract, etc. | 4, 5, 8, 12, 13, 16 |
| Y | MASUKAWA, M. K. et al. Water-in-Water Droplets Selectively Uptake Self-Assembled DNA Nano/Microstructures: a Versatile Method for Purification in DNA Nanotechnology. ChemBioChem. July 2022, vol. 23, e202200240, <doi.org/10.1002/cbic.202200240> abstract, etc. | 1-3, 6, 7, 9-11, 14, 15, 17 |
| A | abstract, etc. | 4, 5, 8, 12, 13, 16 |
| Y | TORRES-BAUTISTA, A. et al. Characterization and optimization of polymer-polymer aqueous two-phase systems for the isolation and purification of CaCo2 cell-derived exosomes. PLOS ONE. September 2022, vol. 17, no. 9, e0273243, <https://doi.org/10.1371/journal.pone.0273243> abstract, table 2, etc. | 1-3, 6, 7, 9-11, 14, 15, 17 |
| A | abstract, table 2, etc. | 4, 5, 8, 12, 13, 16 |

Form PCT/ISA/210 (second sheet) (January 2015)

## INTERNATIONAL SEARCH REPORT

International application No.

**PCT/JP2023/037489**

---

**Box No. I**     **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

    a.   ☑   forming part of the international application as filed:

             ☑   in the form of an Annex C/ST.25 text file.

             ☐   on paper or in the form of an image file.

    b.   ☐   furnished together with the international application under PCT Rule 13*ter*.1(a) for the purposes of international search only in the form of an Annex C/ST.25 text file.

    c.   ☐   furnished subsequent to the international filing date for the purposes of international search only:

             ☐   in the form of an Annex C/ST.25 text file (Rule 13*ter*.1(a)).

             ☐   on paper or in the form of an image file (Rule 13*ter*.1(b) and Administrative Instructions, Section 713).

2.   ☐   In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    "In the form of an Annex C/ST.25 text file" above should be understood as "in ST. 26 format".

---

Form PCT/ISA/210 (continuation of first sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018181488 A
- JP 2022 A
- JP 031760 A
- JP 2022031760 A
- WO 2016047068 A

### Non-patent literature cited in the description

- **ARLYNE B. SIMON**. Aqueous two-phase systems enable multiplexing of homogeneous immunoassay. *TECHNOLOGY*, May 2014, vol. 2 (2), 176-184
- **SUWANNARANGSEE, S** ; **MOULIS, C.** ; **POTOCKI-VERONESE, G** ; **MONSAN, P** ; **REMAUD-SIMEON, M** ; **CHULALAKSANANUKUL, W**. Search for a dextransucrase minimal motif involved in dextran binding. *FEBS Lett*, 2007, vol. 581 (24), 4675-80
- **UENO, H** ; **KATO, M** ; **MINAGAWA, Y.** ; **HIROSE, Y** ; **NOJI, H**. Elucidation and control of low and high active populations of alkaline phosphatase molecules for quantitative digital bioassay.. *Protein Sci*, 2021, vol. 30 (8), 1628-1639
- **KELLNER, M. J.** ; **KOOB, J. G.** ; **GOOTENBERG, J. S** ; **ABUDAYYEH, O. O.** ; **ZHANG, F.** SHERLOCK: nucleic acid detection with CRISPR nucleases. *Nat Protoc*, 2019, vol. 14 (10), 2986-3012
- **GOOTENBERG, J. S** ; **ABUDAYYEH, O. O** ; **KELLNER, M. J** ; **JOUNG, J.** ; **COLLINS, J. J** ; **ZHANG, F**. Multiplexed and portable nucleic acid detection platform with Cas13, Cas12a, and Csm6. *Science*, 2018, vol. 360 (6387), 439-444
- **NAKATANI, N.;** ; **SAKUTA, H.** ; ; **HAYASHI, M** ; **TANAKA, S** ; **TAKIGUCHI, K** ; **TSUMOTO, K** ; **YOSHIKAWA, K**. Specific Spatial Localization of Actin and DNA in a Water/Water Microdroplet: Self-Emergence of a Cell-Like Structure.. *Chembiochem*, 2018, vol. 19 (13), 1370-1374
- **WU, S. C.** ; **WANG, C** ; ; **CHIN, J** ; **WONG, S. L**. A bio-coupling approach using a dextran-binding domain to immobilize an engineered streptavidin to Sephadex for easy preparation of affinity matrix.. *Sci Rep*, 2019, vol. 9 (1), 3359
- **KELLNER, M. J.** ; ; **KOOB, J. G** ; **GOOTENBERG, J. S** ; **ABUDAYYEH, O. O** ; **ZHANG, F**. SHERLOCK: nucleic acid detection with CRISPR nucleases. *Nat Protoc*, 2019, vol. 14 (10), 2986-3012

- **LIU, T. Y.** ; ; **KNOTT, G. J.** ; **SMOCK, D. C. J** ; **DESMARAIS, J. J.** ; **SON, S.** ; **BHUIYA, A.** ; **JAKHANWAL, S** ; **PRYWES, N** ; **AGRAWAL, S.** ; **DERBY, M. D. D.** Accelerated RNA detection using tandem CRISPR nucleases. *Nat Chem Biol*, 2021, vol. 17 (9), 982-988
- **MAHAS, A** ; **WANG, Q. C** ; **MARSIC, T.** ; **MAHFOUZ, M. M.** A Novel Miniature CRISPR- Cas13 System for SARS-CoV-2 Diagnostics.. *Acs Synthetic Biology*, 2021, vol. 10 (10), 2541-2551
- **TIAN, T.** ; **SHU, B.** ; **JIANG, Y.** ; **YE, M.** ; **LIU, L** ; **GUO, Z** ; **HAN, Z** ; **WANG, Z** ; **ZHOU, X**. An Ultralocalized Cas13a Assay Enables Universal and Nucleic Acid Amplification-Free Single-Molecule RNA Diagnostics. *ACS Nano*, 2021, vol. 15 (1), 1167-1178
- **SHINODA, H.** ; **TAGUCHI, Y** ; **NAKAGAWA, R** ; **MAKINO, A** ; **OKAZAKI, S** ; **NAKANO, M.** ; **MURAMOTO, Y** ; **TAKAHASHI, C** ; **TAKAHASHI, I.** ; **ANDO, J**. Amplification-free RNA detection with CRISPR-Cas13. *Commun Biol*, 2021, vol. 4 (1), 476
- **SHINODA, H.** ; **IIDA, T** ; **MAKINO, A** ; **YOSHIMURA, M** ; **ISHIKAWA, J** ; **ANDO, J.** ; **MURAI, K** ; **SUGIYAMA, K** ; **MURAMOTO, Y** ; **NAKANO, M**. Automated amplification-free digital RNA detection platform for rapid and sensitive SARS-CoV-2 diagnosis.. *Commun Biol*, 2022, vol. 5 (1), 473
- **MASTIANI, M.** ; **SEO, S** ; **JIMENEZ, S. M.** ; **PETROZZI, N.** ; **KIM, M. M**. Flow regime mapping of aqueous two-phase system droplets in flow-focusing geometries. *Colloid Surface A*, 2017, vol. 531, 111-120
- **ZHANG, Q. Q.** ; **CHEN, J. Q.** ; **GAI, H. W.** High-throughput-generating water-in-water droplet for monodisperse biocompatible particle synthesis.. *J Mater Sci*, 2019, vol. 54 (24), 14905-14913
- **ZHOU, C.** ; **ZHU, P.** ; **HAN, X** ; **SHI, R.** ; **TIAN, Y** ; **WANG, L**. Microfluidic generation of ATPS droplets by transient double emulsion technique.. *Lab Chip*, 2021, vol. 21 (14), 2684-2690